(19) Europäisches Patentamt
European Patent Office
Office européen des brevets

(11) EP 4 503 053 A1

(12) **EUROPEAN PATENT APPLICATION**
published in accordance with Art. 153(4) EPC

(43) Date of publication:
05.02.2025 Bulletin 2025/06

(21) Application number: 23778416.0

(22) Date of filing: 30.03.2023

(51) International Patent Classification (IPC):
*G16H 50/70* (2018.01)

(52) Cooperative Patent Classification (CPC):
G16H 50/70

(86) International application number:
PCT/CN2023/085278

(87) International publication number:
WO 2023/186051 (05.10.2023 Gazette 2023/40)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 31.03.2022 CN 202210346832
20.04.2022 CN 202210416282
20.04.2022 CN 202210416283

(71) Applicant: Shenzhen Dymind Biotechnology Co.,
Ltd
Shenzhen, Guangdong 518107 (CN)

(72) Inventors:
• WANG, Yuting
Shenzhen, Guangdong 518000 (CN)
• FANG, Jianwei
Shenzhen, Guangdong 518000 (CN)
• HUO, Ziling
Shenzhen, Guangdong 518000 (CN)

(74) Representative: Herrero & Asociados, S.L.
Edificio Aqua - Agustín de Foxá, 4-10
28036 Madrid (ES)

(54) **AUXILIARY DIAGNOSIS METHOD AND APPARATUS, AND CONSTRUCTION APPARATUS, ANALYSIS APPARATUS AND RELATED PRODUCT**

(57) Disclosed in the embodiments of the present invention are an auxiliary diagnosis method and apparatus, a construction apparatus for an auxiliary diagnosis knowledge graph, a biological sample analysis apparatus, a sample analyzer, and a sample analysis system. The auxiliary diagnosis apparatus comprises: an acquisition module, which acquires an examination parameter corresponding to a biological sample of an object to be subj ected to examination, and a recognition result of a target feature of the biological sample; a similar feature determination module, which searches, according to the recognition result of the target feature, a sample feature library including sample features corresponding to historical diagnosis examples for similar sample feature information corresponding to the recognition result of the target feature; an auxiliary decision-making module, which determines an auxiliary diagnosis result of said object; and an output module, which outputs an auxiliary diagnosis report including the auxiliary diagnosis result and a diagnosis basis which characterizes the auxiliary diagnosis result, wherein the diagnosis basis comprises a sample analysis image in the recognition result that correspondingly represents the target feature is abnormal, and a similar sample image in the similar sample feature information that correspondingly represents the target feature is abnormal.

FIG.13

EP 4 503 053 A1

**Description**

**TECHNICAL FIELD**

The present disclosure relates to the field of medical assistive technologies, and in particular to an auxiliary diagnosis apparatus, a construction apparatus for auxiliary diagnosis knowledge map, a biological sample analysis apparatus, an auxiliary diagnosis method, a sample analyzer, and a sample analysis system.

**BACKGROUND**

As the number of patients with diseases increases, the demand for medical consultations also continues to increase. However, there are too few experienced senior doctors in medical institutions to meet the huge demand for medical consultations. The existing methods for diagnosing diseases mainly rely on doctors' diagnoses based on their personal experience. Specifically, doctors can generate a diagnosis based on the patient's clinical information and test data, combined with their own experience.

However, the overall efficiency of the existing manual diagnosis is relatively low. Therefore, equipment-based auxiliary diagnosis technology has emerged.

**SUMMARY OF THE DISCLOSURE**

Technical problems

In order to solve the existing technical problems, the present disclosure provides an auxiliary diagnosis apparatus, a construction apparatus for auxiliary diagnosis knowledge map, a biological sample analysis apparatus, an auxiliary diagnosis method, a sample analyzer, and a sample analysis system. Technical solution

In the first aspect, the present disclosure provides an auxiliary diagnosis apparatus, including:

an obtaining module, configured to obtain detection parameters corresponding to a biological sample of a to-be-tested object and an identification result of a target feature of the biological sample of the to-be-tested object;

a similar feature determination module, configured to search from a sample feature library containing sample features corresponding to historical diagnosis cases according to the identification result of the target feature, for determining similar sample feature information corresponding to the identification result of the target feature;

an auxiliary decision-making module, configured to determine an auxiliary diagnosis result of the to-be-tested object based on the detection parameters, the identification result of the target feature, and the similar sample feature information; and

an output module, configured to output an auxiliary diagnosis report containing the auxiliary diagnosis result and a diagnosis basis characterizing the auxiliary diagnosis result; wherein the diagnosis basis comprises a sample analysis image characterizing an abnormality of the target feature in the identification result of the target feature and a similar sample image characterizing an abnormality of the target feature in the similar sample feature information.

Beneficial effects

The auxiliary diagnosis apparatus provided by the embodiments present disclosure obtains an auxiliary diagnosis result based on the detection parameters of the to-be-tested object and the identification result of the target feature, matches the similar sample feature information based on the identification result, and outputs an auxiliary diagnosis report including the auxiliary diagnosis result and the diagnosis basis of the auxiliary diagnosis result. The diagnosis basis includes: first feature information corresponding to the identification result of the target feature indicating an abnormality of the target feature, and second feature information corresponding to the similar sample feature information indicating an abnormality of the target feature, where the first feature information may be a sample analysis image to indicate that the target feature is abnormal, and the second feature information may be a similar sample image that indicates that the target feature is abnormal. The auxiliary diagnosis report provides the auxiliary diagnosis result and its diagnosis basis, such that the user can more intuitively obtain auxiliary diagnosis information, and quickly and accurately understand the reliability of the current auxiliary diagnosis result based on the diagnosis basis.

**BRIEF DESCRIPTION OF THE DRAWINGS**

FIG. 1 is a diagram showing an application scenario of an auxiliary diagnosis apparatus according to some embodiments of the present disclosure.

FIG. 2 is a diagram showing an application scenario of an auxiliary diagnosis apparatus according to other embodiments of the present disclosure.

FIG. 3 is a flowchart of an auxiliary diagnosis method according to some embodiments of the present disclosure.

FIG. 4 is a diagram showing the interface of different auxiliary diagnosis reports according to some embodiments of the present disclosure.

FIGS. 5 to 11 are flowcharts of auxiliary diagnosis methods provided in different embodiments.

FIG. 12 is a diagram showing the interface of an auxiliary diagnosis report according to other embodiments of the present disclosure.

FIG. 13 is a structural schematic diagram of an auxiliary diagnosis apparatus according to some embodiments of the present disclosure.

FIG. 14 is a flowchart of a method for constructing an auxiliary diagnosis knowledge map according to some embodiments of the present disclosure.

FIG. 15 is a flowchart of a method for constructing an auxiliary diagnosis knowledge map according to other embodiments of the present disclosure.

FIG. 16 is a flowchart of a method for constructing an auxiliary diagnosis knowledge map according to further other embodiments of the present disclosure.

FIG. 17 is a flowchart of a method for constructing an auxiliary diagnosis knowledge map according to still other embodiments of the present disclosure.

FIG. 18 is a structural schematic diagram of an apparatus for constructing an auxiliary diagnosis knowledge map according to some embodiments of the present disclosure.

FIG. 19 is a flowchart of a method for analyzing biological samples according to some embodiments of the present disclosure.

FIG. 20 is a structural schematic view of an apparatus for analyzing biological samples according to some embodiments of the present disclosure.

## DETAILED DESCRIPTION

The following combined description with the accompanying drawings and specific embodiments further elaborates the technical solution of the present disclosure.

Unless otherwise defined, all technical and scientific terms used herein have the same meaning as commonly understood by one skilled in the technical field of the present disclosure. The terms used herein in the description of the present disclosure are for the purpose of describing specific embodiments only and are not intended to limit the scope of the present disclosure. The terms "and/or" as used herein include any and all combinations of one or more relevant listed items.

In the following description, the expression of "some embodiments", etc. is intended to describe a subset of all possible embodiments. It should be understood that "some embodiments" can be the same subset or different subsets of all possible embodiments, and can be combined with each other without conflict.

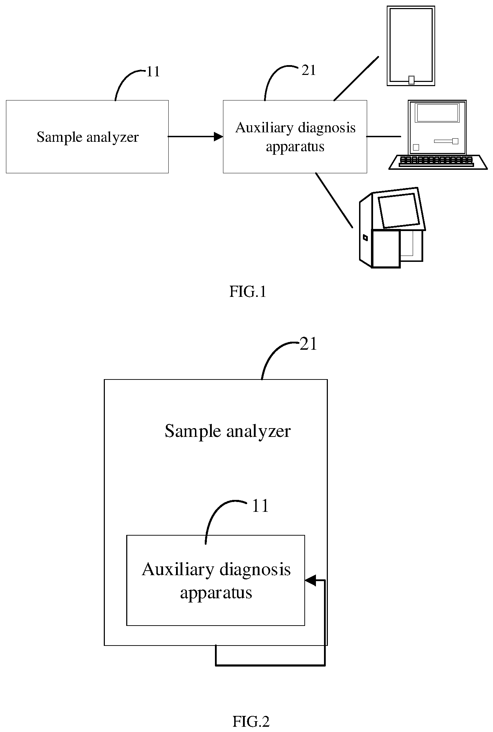

Referring to FIG. 1, FIG. 1 is a diagram showing an application scenario of an auxiliary diagnosis apparatus according to some embodiments of the present disclosure, where the auxiliary diagnosis apparatus 11 may refer to a computer program product based on a computer program process to implement an auxiliary diagnosis function (i.e., to implement the auxiliary diagnosis method described in any of the following embodiments of the present disclosure), such as various types of application programs; the auxiliary diagnosis apparatus 11 may refer to an auxiliary diagnosis device loaded with a corresponding computer program product to realize the auxiliary diagnosis function (i.e., to realize the auxiliary diagnosis method described in any of the following embodiments of the present disclosure), such as various types of intelligent devices with storage and computing capabilities.

In practical applications, in a case where the auxiliary diagnosis apparatus 11 refers to a auxiliary diagnosis device loaded with a corresponding computer program product, it may be a physically separate intelligent device itself or integrated with a known intelligent device. As shown in FIG. 1, the auxiliary diagnosis apparatus 11 is an intelligent device physically separated from a sample analyzer 21, such as a smartphone, personal computer, medical diagnosis instrument, cloud server, etc. Referring to FIG. 2, the auxiliary diagnosis apparatus 11 is integrated with the sample analyzer 21, such as a sample analyzer loaded with a corresponding computer program product.

The auxiliary diagnosis apparatus 11 may be configured to communicate with an output interface of an application system within the sample analyzer 21 that performs detection and analysis on a sample, for obtaining sample detection data obtained after the detection and analysis of a biological sample of a to-be-tested object from the sample analyzer 21. The sample analyzer 21 may refer to a device for performing intelligent detection and analysis on collected biological samples. The biological sample may be a sample taken from the body of the to-be-tested object and containing various biological cell information or other biological information, such as a blood sample, urine sample, or other body fluid (pleural effusion, cerebrospinal fluid, serous cavity effusion, synovial fluid) sample. The type of biological cell may be at least one of the groups composed of neutrophils, lymphocytes, monocytes, eosinophils, and basophils; it may be immature granulocytes, tumor cells, lymphoblasts, plasma cells, atypical lymphocytes, proerythroblasts, basophilic erythroblasts and polychromatic erythroblasts, orthochromatic erythroblasts, proterythroblasts, basterythroblasts, polychromatic megakaryocytes and nucleated erythrocytes selected from orthochromatic megakaryocytes and macrocytes.

The auxiliary diagnosis apparatus 11 obtains sample testing data from the sample analyzer 21 obtained by testing and analyzing the biological sample, uses the sample testing data in conjunction with clinical information about the to-be-tested object and a case library formed based on historical diagnosis records to determine abnormal features of the biological sample, and output an auxiliary diagnosis report based on the identified abnormal features of the biological sample. The auxiliary diagnosis report is configured to provide auxiliary diagnosis category information determined based on the abnormal features in the biological sample and a diagnosis basis for obtaining the auxiliary diagnosis category information. In this way, by viewing the auxiliary diagnosis report, a user may more intuitively understand the current possible or potential medical conditions of the to-be-tested object. Further, in a case where the presence of an abnormality is determined, the basis for the decision can be known through the diagnosis basis provided in the auxiliary diagnosis report, without relying on the personal level of experience of the testing physician, such that accurate auxiliary diagnosis results can be obtained. In this way, on the one hand, test efficiency and accuracy may be improved. On the other hand, the diagnosis conclusions are more interpretable, where the sample testing images and sample testing parameters obtained by the sample analyzer can be converted into a descriptive auxiliary diagnosis report to assist the test physician in reducing the workload of screening test data, and further, the results in the auxiliary diagnosis report can better match the hierarchical diagnosis and treatment policy.

Optionally, the auxiliary diagnosis apparatus 11 may determine the auxiliary diagnosis category information by comprehensively considering various data of the to-be-tested object, such as clinical information data that reflects individual differences of the to-be-tested object, generally including gender, age, medical history, etc. The auxiliary diagnosis apparatus 11 may utilize clinical information data in multiple stages. One stage is that the sample analyzer 21 considers the clinical information data of the to-be-tested object to calibrate the obtained sample test data during the process of testing and analyzing the biological sample of the to-be-tested object. Another stage is that the auxiliary diagnosis apparatus 11 directly obtains the clinical information data of the to-be-tested object, and further comprehensively considers the clinical information data of the to-be-tested object to calibrate the auxiliary diagnosis category information obtained, in the process of determining the auxiliary diagnosis category information based on a sample analysis image, and first abnormal feature information and second abnormal feature information identified. In some embodiments, the use of the clinical information data of the to-be-tested object by the auxiliary diagnosis apparatus 11 includes the above-mentioned second stage. The auxiliary diagnosis apparatus 11 is communicatively connected to a laboratory information system (LIS), which generally includes application terminals arranged at different locations such as a reception desk and a laboratory of a hospital, which may be configured to receive test data, enter and save patient test information, and assist the hospital in information management. The auxiliary diagnosis apparatus 11 may directly obtain the clinical information data of a specified category of the to-be-tested object from the laboratory information system.

To facilitate understanding of the technical implementation of the auxiliary diagnosis apparatus 11 provided in the embodiments of the present disclosure, specific examples in the description are mainly explained in detail using the biological sample as a blood sample, and the sample analyzer 21 refers to a blood cell analyzer. A known blood cell analyzer is a typical application of flow cytometry. The impedance channel for cell counting utilizes the Coulter impedance principle, the channels for hemoglobin concentration determination utilizes the colorimetric method principle, and the optical channels for higher-end blood cell analyzers such as those used for white blood cell classification, identification of reticulocytes, identification of nucleated red blood cells, identification of basophils, identification of low-value platelets, and identification of primitive cells utilizes the laser scattering and nucleic acid fluorescent staining techniques. The goal of these techniques is to convert the biological features of blood cells, such as size, complexity of cell contents, and nucleic acid content, into electrical pulse signals. These electrical pulse signals are the raw data collected by the blood cell analyzer. By analyzing the collected electrical pulse signals, sample analysis images and numerical sample testing parameters that characterize the features of blood cells can be output. However, it should be noted that although the description of the embodiments of the present disclosure uses sample testing data to describe the testing and analysis data of a blood sample as an example, this shall not constitute a limitation of the scope of the present disclosure. For example, in other embodiments, the sample analyzer 21 may be a biochemical analyzer. The biochemical analyzer analyzes different biological samples according to different testing requirements. For example, for liver function testing, the biochemical analyzer analyzes the biological sample of the to-be-tested object for alanine aminotransferase (ALT/GPT), aspartate aminotransferase (AST/GOT), alkaline phosphatase (ALP), total bilirubin (T.BIL), direct bilirubin (D.BIL), total protein (TP), and albumin (ALB). For kidney function testing, the biochemical analyzer analyzes the biological sample of the to-be-tested object for urea nitrogen (BUN), creatinine (Cre), carbon dioxide binding capacity ($CO_2$), and uric acid (UA). Similarly, for lipid testing, the biochemical analyzer analyzes the biological sample of the to-be-tested object for total cholesterol (CHO), triglycerides (TG), high-density lipoprotein cholesterol (HDL-C), and low-density lipoprotein cholesterol (LDL-C). For blood glucose testing, the biochemical analyzer analyzes glucose (GLU) in the biological sample. In other embodiments, the sample analyzer 21 may further be an immunoassay analyzer for obtaining sample test data for analysis of tumor markers, features related to thyroid function, features related to reproduction/endocrinology, features related to cardiovascular diseases and/or features related to congenital diseases in the biological sample. In other embodiments, the sample testing data of the to-be-tested object may be obtained from multiple different sample analyzers 21, such as a blood cell analyzer, a biochemistry analyzer, and an immunoassay analyzer. Accordingly, the sample testing data obtained by the auxiliary diagnosis apparatus 11 may include one or more of blood cell analysis data, biochemistry analysis data, and immunoassay analysis data.

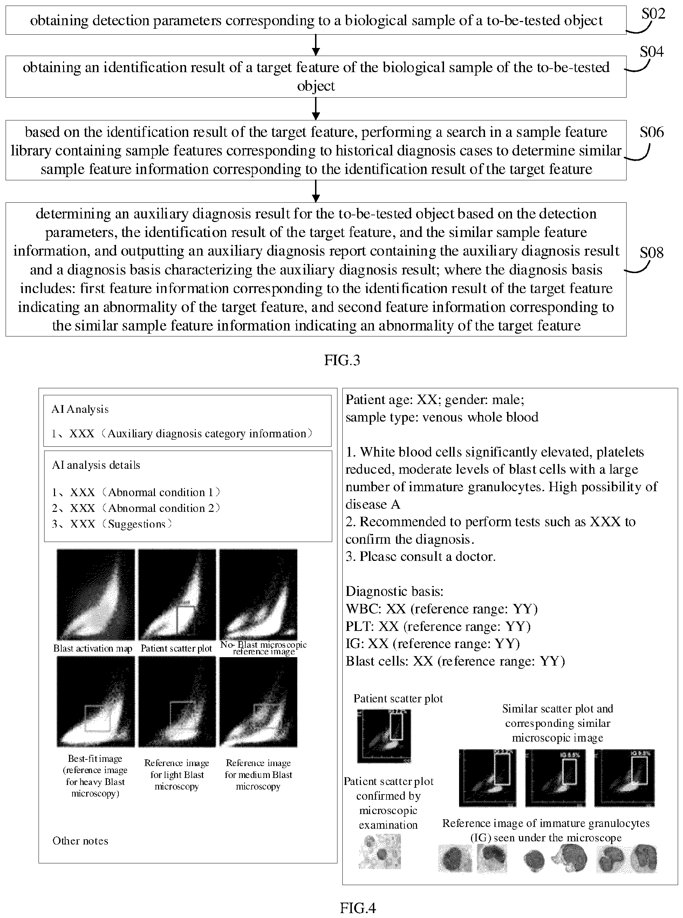

Referring to FIG. 3, FIG. 3 is a flowchart of an auxiliary diagnosis method according to some embodiments of the present disclosure. The method can be applied to the auxiliary diagnosis apparatus in FIG. 1 or 2, and the auxiliary diagnosis method includes, but is not limited to, operations at blocks 502, S04, S06 and S08, as described below.

At block S02: obtaining detection parameters corresponding to a biological sample of a to-be-tested object.

The to-be-tested object refers to the owner of the biological sample. Taking the biological sample as a human blood sample as an example, the to-be-tested object usually refers to the patient who provides the blood sample. Different objects to be tested have different ages, genders, types of test samples, and past medical histories, and different cells in the blood samples have different features. In an optional specific example, the method further includes: obtaining clinical information data of the to-be-tested object, where the clinical information data includes age, gender, type of the biological sample, past medical history of the to-be-tested object, and microscopic examination results of the to-be-tested object. In an optional specific example, the obtaining the detection parameters of the biological sample includes: obtaining sample testing data output by a sample analyzer that tests the biological sample; for a blood cell analyzer, the sample analysis data required to obtain an auxiliary diagnosis result includes the detection parameters of the biological sample. The detection parameters include reporting parameters, instrument performance parameters of the sample analyzer, and research parameters of the sample analyzer. The reporting parameters include information on the counting of cell components in samples such as white blood cell count, red blood cell count, and platelet count. The research parameters include nucleated red blood cell count (NRBC) and immature platelet fraction (IPF), etc., and the instrument performance parameters refer to parameters of the pore voltage data of the testing channel of the sample analyzer that reflect the current state of the sample analyzer, such as the pulse feature signal parameters of the voltage change when the pore is blocked. In some embodiments, the reporting parameters of the to-be-tested object refer to test report parameters corresponding to the output of the biological sample under different sample analyzers. Taking the detection and analysis of a blood sample under a blood cell analyzer as an example, the corresponding test report parameters may be one or more selected from red blood cells (RBC), hemoglobin (HGB), hematocrit (HCT), mean corpuscular volume (MCV), mean corpuscular hemoglobin content (MCH), mean corpuscular hemoglobin concentration (MCHC), white blood cells (WBC), red blood cell distribution width (RDW-CV), red blood cell distribution width standard deviation (RDW-SD), platelets (PLT), and reticulocytes (RET). The test report parameters are not exactly the same depending on the testing mode of the blood cell analyzer. The testing modes of the blood cell analyzer include a CD testing mode and a CDR testing mode. Among them, the CD detection mode is configured to obtain a complete blood count and differential leukocyte (white blood cell) count (CBC+DIFF), and the CDR detection mode is configured to obtain a complete blood count and differential leukocyte count and test reticulocyte detection (CBC+DIFF+RET). The test report parameters include the reporting parameters obtained in one testing mode of the blood cell analyzer or the reporting parameters obtained in multiple testing modes.

At block S04: obtaining an identification result of a target feature of the biological sample of the to-be-tested object.

The target feature in the biological sample refers to feature, in biological information features contained in the biological sample, that is relevant to testing requirements. For example, in the case of a blood sample, the biological information features include blood cell features, which may be different types of cells corresponding to healthy or unhealthy features presented in the biological sample. For example, the biological sample may contain abnormal cell types, cell quantity features, cell size features, cell composition ratio features, cell content features, nucleic acid content features, etc. The target feature is biological information features determined according to the testing requirements of different biological samples. The target feature refers to one or more cell features determined according to the testing requirements of different biological samples. For example, taking the analysis of white blood cells in a blood sample as an example, the target feature may include white blood cell count features or white blood cell size features, where the white blood cells may be further classified, and the white blood cell size features may include size feature of a specific type of white blood cell, such as an immature granulocyte.

At block S06: based on the identification result of the target feature, performing a search in a sample feature library containing sample features corresponding to historical diagnosis cases to determine similar sample feature information corresponding to the identification result of the target feature.

The historical diagnosis cases refer to the cases corresponding to tested object. Each historical diagnosis case includes clinical information, test information, and diagnosis result corresponding to a corresponding tested object. The test information includes the sample analysis data obtained by a corresponding sample analyzer for the biological sample corresponding to the tested object, such as the detection parameters, etc., as well as the identification result obtained by identifying the target feature in the biological sample, and further includes the sample feature information corresponding to the biological sample of the tested object. The diagnosis result is the disease category corresponding to the tested object. It should be noted that the disease category further includes Normal category, indicating that the corresponding tested object is normal with respect to a certain disease type and does not suffer from that disease type. The sample feature library refers to a library that stores the sample feature information of historical diagnosis cases. The sample feature information includes sample analysis feature information obtained by the sample analyzer itself, such as detection pulse signal features and sample analysis image features obtained by the sample analyzer when testing the biological sample, and instrument status signal features of the sample analyzer. The instrument status signal features of the sample analyzer may be configured to determine whether the sample analyzer that is testing the biological sample is operating normally, so as to assess the accuracy of the subsequent auxiliary diagnosis results. When the instrument status signal features indicate that the sample analyzer is operating abnormally, it means that the auxiliary diagnosis results obtained based on the sample analysis data from the sample analyzer in an abnormal operating state are also inaccurate. In addition, the sample feature information further includes reference feature information obtained by the sample analyzer, which serves as a reference for the sample analyzer to analyze the biological sample. The reference feature information may be information obtained by the sample analyzer from other instruments or information pre-set in the sample analyzer. In some embodiments, the reference feature information includes microscopic image feature information representing the cell morphology in the biological sample. The microscopic image may be a microscopic image stored in a microscopic image library pre-established in the biological sample analyzer or a microscopic image obtained by the biological sample analyzer from a microscopic apparatus (e.g., a film reader).

A similar sample feature information that is similar to the identification result of the target feature refers to the sample feature information corresponding to the target feature obtained from the sample feature library for the biological sample of the tested object, i.e., the sample feature information that can reflect the target feature. The identification result of the target feature usually mainly refers to an abnormal feature that can reflect the unhealthy state of the to-be-tested object; the similar sample feature information may include: normal sample feature information corresponding to the target feature that is normal, and abnormal sample feature information corresponding to the target feature that is abnormal. In addition, the sample feature information corresponding to the target feature in the biological sample of the tested object may include a microscopic image library formed based on the microscopic image of the tested object, and the similar sample feature information may include a similar microscopic image corresponding to the abnormal target feature in the microscopic image library. In some embodiments, the similar microscopic image includes microscopic images that respectively characterize different abnormality degrees of the abnormal sample image.

At block S08: determining an auxiliary diagnosis result for the to-be-tested object based on the detection parameters, the identification result of the target feature, and the similar sample feature information, and outputting an auxiliary diagnosis report containing the auxiliary diagnosis result and a diagnosis basis characterizing the auxiliary diagnosis result; where the diagnosis basis includes: first feature information corresponding to the identification result of the target feature indicating an abnormality of the target feature, and second feature information corresponding to the similar sample feature information indicating an abnormality of the target feature.

Optionally, the auxiliary diagnosis apparatus may further obtain clinical diagnosis data of the to-be-tested object, and comprehensively obtain the auxiliary diagnosis result of the to-be-tested object and the corresponding diagnosis basis based on the datasets of the clinical information of the to-be-tested object, the detection parameters, the identification result of the target feature, and the similar sample feature information. Specifically, the first feature information may be a sample analysis image that marks the abnormality of the target feature, and the second feature information may be a similar sample image that marks the abnormality of the target feature. The auxiliary diagnosis apparatus determines the auxiliary diagnosis result by matching the above sets of data of the to-be-tested object to a predetermined disease diagnosis criterion rule, and a disease type corresponding to the disease diagnosis criterion rule with the highest matching degree is taken as the auxiliary diagnosis result. The disease diagnosis criterion rule may be obtained by mining the historical diagnosis cases of the tested object through an AI data mining model, or it may be obtained based on an expert consensus database. The auxiliary diagnosis result includes the disease type that the to-be-tested object may suffer from.

Referring to FIG. 4, FIG. 4 is a diagram showing the interface of different auxiliary diagnosis reports according to some embodiments of the present disclosure, including the auxiliary diagnosis result and diagnosis basis. The auxiliary diagnosis result includes information about the disease type or abnormality that may be diagnosed in the to-be-tested object. The auxiliary diagnosis result may be as shown in FIG. 4: white blood cells significantly elevated, platelets reduced, and moderate number of primitive cells with a large number of immature granulocytes. The likelihood of disease A is high, and it is recommended that a test of XXX be performed to confirm the diagnosis, taking into account the clinical situation. That is, the auxiliary diagnosis report further includes recommended information based on the auxiliary diagnosis result. The diagnosis basis includes the values of the reported parameters and their corresponding reference values related to the determination of the disease type in the diagnosis result, as well as a sample analysis image of the to-be-tested object that includes the identification result marked with target feature, such as a scatter plot of the patient, and scatter plots of other patients marked with target feature for an abnormality of the target feature and a corresponding similar sample image and microscopic image, where the similar sample image may be a sample analysis image of a historical diagnosis case confirmed by microscopic examination, and the microscopic image includes microscopic images of the target feature with different abnormality degrees and microscopic images of the target feature with a normality. According to the auxiliary diagnosis report, the user can compare the patient's sample image with similar sample images of other patients and abnormal identification information in similar microscopic images, so as to obtain not only the auxiliary diagnosis result containing the auxiliary diagnosis category information determined by comprehensive and multi-faceted information, but also make full use of the computer intelligence to identify the sample analysis image to mine more in-depth image information containing biological information features, thereby improving the reliability, accuracy, and efficiency of the auxiliary diagnosis results. Moreover, the sample analysis image and similar sample image output in the auxiliary diagnosis report, that can visually represent the abnormality of the target feature, may facilitate the doctor to quickly verify the accuracy of the current auxiliary diagnosis category information, so as to consider whether to adopt the current auxiliary diagnosis category information, i.e., the auxiliary diagnosis report is highly interpretable.

In some embodiments, the S02: obtaining detection parameters corresponding to a biological sample of a to-be-tested object includes at least one of the following:

> obtaining test report parameters or abnormal sample parameters output by the sample analyzer that performs a test on the biological sample of the to-be-tested object;
> obtaining research parameters of the biological sample of the to-be-tested object; and
> obtaining status parameters of a testing device that performs the test on the biological sample of the to-be-tested object.

For a blood cell analyzer, the detection parameters of the biological sample may include reporting parameters, instrument performance parameters of the sample analyzer, and research parameters of the sample analyzer. The reporting parameters include information on the counting of cell components in samples such as white blood cell count, red blood cell count, and platelet count. The research parameters include nucleated red blood cell count (NRBC) and immature platelet fraction (IPF), etc., and the instrument performance parameters refer to parameters of the pore voltage data of the testing channel of the sample analyzer that reflect the current state of the sample analyzer, such as the pulse feature signal parameters of the voltage change when the pore is blocked. In some embodiments, the reporting parameters of the to-be-tested object include corresponding reporting parameters of the biological sample under different sample analyzers. Taking the detection and analysis of a blood sample under a blood cell analyzer as an example, the corresponding test report parameters may be one or more selected from red blood cells (RBC), hemoglobin (HGB), hematocrit (HCT), mean corpuscular volume (MCV), mean corpuscular hemoglobin content (MCH ), mean corpuscular hemoglobin concentration (MCHC), white blood cells (WBC), red blood cell distribution width (RDW-CV), red blood cell distribution width standard deviation (RDW-SD), platelets (PLT), and reticulocytes (RET).

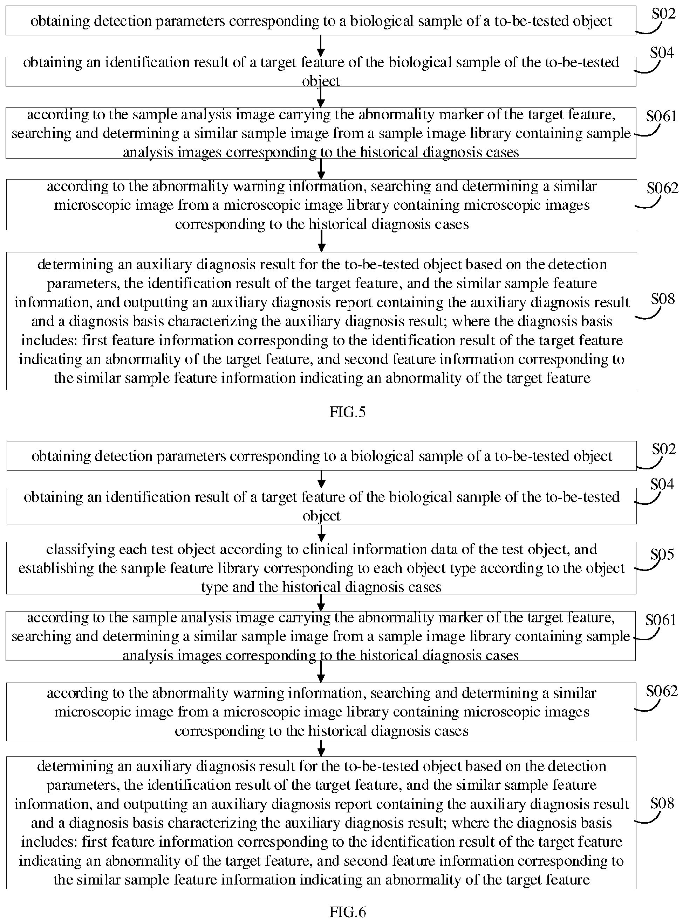

Referring to FIG. 5, in some embodiments, the S04: obtaining an identification result of a target feature of the biological sample of the to-be-tested object includes: obtaining abnormality warning information characterizing an abnormality of the target feature, and a sample analysis image carrying an abnormality marker of the target feature.

The abnormality warning information is information indicating that the target feature in the biological sample of the to-be-tested object is abnormal, such as IG-positive warning information, Blast abnormality warning information, etc.

Continuing to refer to FIG. 5, the S06: based on the identification result of the target feature, performing a search in a sample feature library containing sample features corresponding to historical diagnosis cases to determine similar sample feature information corresponding to the identification result of the target feature, further includes operations at blocks S061 and S062, as described in detail below.

At block S061: according to the sample analysis image carrying the abnormality marker of the target feature, searching and determining a similar sample image from a sample image library containing sample analysis images corresponding to the historical diagnosis cases.

At block S062: according to the abnormality warning information, searching and determining a similar microscopic image from a microscopic image library containing microscopic images corresponding to the historical diagnosis cases.

Optionally, the sample image library includes sample analysis images, corresponding to the historical diagnosis cases, that are confirmed by microscopy. In the embodiments, a similar sample image matching the target identification result is searched from the sample image library in the sample feature library based on the identification result of the target feature. The abnormality marker is configured to indicate the category and location of the target feature in the sample analysis image containing the target feature corresponding to the biological sample of the to-be-tested object. The sample analysis image carrying the abnormality marker refers to a sample analysis image in which the target feature is highlighted by the marker. The sample analysis image of the to-be-tested object refers to image data contained in the sample analysis data obtained by the biological sample analyzer for the biological sample of the to-be-tested object, and the biological sample analyzer includes but is not limited to one or more of a blood cell analyzer, a biochemistry analyzer, and an immunoassay analyzer. Specifically, the similar sample image may be obtained based on the sample analysis image with the abnormality marker according to a similar image recommendation algorithm, and a direct match can be made with the abnormality warning information to obtain the corresponding similar microscopic image, i.e., the microscopic image matched with the abnormality warning information. For example, based on the IG positive warning information, a microscopic image corresponding to abnormal immature granulocytes can be matched.

Continuing to refer to FIG. 5, in some embodiments, the S06, based on the identification result of the target feature, performing a search in a sample feature library containing sample features corresponding to historical diagnosis cases to determine similar sample feature information corresponding to the identification result of the target feature, includes: according to the abnormality warning information in the identification result of the target feature, searching from the microscopic image library containing corresponding microscopic images corresponding to the historical diagnosis cases to determine a reference microscopic image representing an abnormality degree of the target feature corresponding to the abnormality warning information. Then, in the S08, the auxiliary diagnosis basis further includes third feature information representing the abnormality degree of the target feature. The third feature information may be a plurality of microscopic images representing different abnormality degrees of the target feature.

Taking a biological sample such as blood cells as an example, the analysis data of the biological sample (in different embodiments, the analysis data of the biological sample and the sample analysis data collectively refer to the image-type and value-type detection parameters obtained by detecting the biological sample) includes a biological sample analysis image. The biological sample analysis image includes at least one of the following: blood cell histogram, blood cell scatter plot, pulse signal waveform diagram, pulse signal feature map, thromboelastogram, and biochemical or immune response curve. Among the detection parameters corresponding to blood cells, blood cell numerical analysis data includes: blood test report parameters, flag alarm parameters, etc.; the blood test report parameters include red blood cells (RBC), hemoglobin (HGB), hematocrit (HCT), mean corpuscular volume (MCV), mean corpuscular hemoglobin content (MCH), white blood cells (WBC), mean corpuscular hemoglobin concentration (MCHC), red blood cell distribution width (RDW- CV), red blood cell distribution width standard deviation (RDW-SD), platelets (PLT), and reticulocytes (RET). The blood cell analysis image may include a histogram corresponding to different blood cells in the blood, a scatter plot characterizing the distribution features of each type of cell, a pulse signal waveform diagram, and a pulse signal feature map. Among them, the blood cell histogram and blood cell scatter plot may be, for example, a platelet histogram, a red blood cell histogram, a white blood cell histogram, a white blood cell DIFF scatter plot, a RET scatter plot, histograms formed by pulse features such as peak height, leading peak width, trailing peak width, and half-peak width of the pulse signal (e.g., the histograms of RBC, PLT, and WBC formed by pulse height), and scatter plots in two, three, or even multiple dimensions formed by the pulse features of several dimensions (e.g., the DIFF three-dimensional scatter dataset formed by the peak heights of the SFL, SSC, and FSC three-channel pulse signals), and two-dimensional scatter plot projections formed by three-dimensional and high-dimensional scatter data. The pulse signal waveform diagram may be, for example, a waveform diagram formed from raw pulse signals collected by a blood cell analyzer during testing of a blood sample, or a pulse signal waveform diagram formed from pulse signals screened by a pulse identification algorithm. The pulse signal feature map may be, for example, vector feature maps formed from pulse features values such as peak height, leading peak width, trailing peak width, and half-peak width of the pulse signal. The blood cell analysis image may be obtained by extracting the numerical test report parameters from the sample analyzer.

Different blood cell analysis data correspond to the features of different cells in the blood, which can characterize whether there are abnormalities in the content and distribution of the corresponding cells in the blood. Therefore, the blood cell analysis data carries the corresponding target feature in the biological sample. Therefore, the blood cell analysis data related to the testing requirements can be characterized and identified to obtain the identification result of the target feature of the biological sample of the to-be-tested object.

In the embodiments, the identification result of the target feature is based on the identification result of the target feature obtained by feature extraction and identification of the blood cell analysis graph. Accordingly, the identification result of the target feature is a blood cell analysis image carrying an abnormality marker. The similar sample feature information may refer to a similar image recommendation algorithm that searches and determines the similar sample image that matches a blood cell analysis image carrying an abnormality marker in the sample image library containing sample images corresponding to historical diagnosis cases. Specifically, the recommendation of similar images may be based on a hash algorithm. After obtaining the similar sample image, according to information on the target feature characterized in the similar sample image, a microscopic image corresponding to the target feature is searched and determined from the microscopic image library containing microscopic images corresponding to historical diagnosis cases as the similar microscopic image.

In some embodiments, the S04: obtaining an identification result of a target feature of the biological sample of the to-be-tested object further includes: obtaining an activation map that is determined based on the abnormality warning information and that prominently displays an abnormal feature region. The activation map is obtained by superimposing a

8

heat map obtained based on a feature map of the sample analysis image on the sample analysis image; where the heat map characterizes a decision-making influence of each cell region of the sample analysis image on determining the first abnormal feature information. The sample analysis image carries a positioning marker, which is formed by a highlighted region based on the activation map and is configured to indicate the location of the identification result of the target feature in the sample analysis image.

Specifically, an image analysis model may be applied to extract features and identify the sample analysis image of the biological sample of the to-be-tested object, obtain the sample analysis image carrying the abnormality marker of the target feature and the abnormality warning information, and then highlight the abnormal feature region, on which the abnormality warning information is based, on the sample analysis image input by the image analysis model, thereby obtaining the activation map.

For example, in some embodiments, the image analysis model may be a classification neural network model, which may include a pooling layer, and may be adopted with visual feature visualization algorithms such as Guided-Backpropagation, Grad-CAM, Score-CAM, and Group-CAM. The image analysis model may utilize a spatial correspondence between the feature map output by the last convolutional layer and the original image to mark the image region that determines the abnormality warning information as the region of greater interest of the neural network model on the original image, and output it synchronously with the abnormality warning information.

The heat map may be a feature map obtained from a feature extraction network in the neural network model. By calculating the weight values of each channel of the feature map output from the feature extraction network to a classification output layer, a machine vision visualization algorithm is applied to linearly weight and sum the image features output by each channel based on the weight values, and display a corresponding image based on the result of the linear weighted sum, that is, the image of the region of interest corresponding to the classification result output by the neural network model. In a specific example, the machine vision visualization algorithm refers to the Grad-CAM algorithm. The Grad-CAM algorithm utilizes a backpropagation between adjacent network layers in the neural network model to achieve the features of training and learning, and can be compatible with different types of neural network models (e.g., deep convolutional neural networks VGG and residual neural networks ResNet) without retraining to obtain a heat map. In the embodiments, the Grad-CAM algorithm calculates the weight values of each channel of the feature map by using a backpropagation gradient of a classification convolutional neural network (CNN), as shown in the following Equation 1:

$$a_k^c = \frac{1}{Z} \sum_i \sum_j \frac{\partial y^c}{\partial A_{ij}^k} \qquad \text{(Equation 1)}$$

In Equation 1, c represents a category, $y^c$ is the logits corresponding to the category (i.e., the value before it is output through the classification layer (Softmax layer)), A represents a feature map output by the convolution (image features output by the feature extraction network), k represents a channel of the feature map, $ij$ represents the horizontal and vertical coordinates of the feature map, and Z represents the size (i.e., length times width) of the feature map. The Grad-CAM algorithm is based on the process of obtaining a heat map from an input image, which is equivalent to finding the mean of the gradient on the feature map of the input image, implementing a global average pooling operation, and then obtaining the weight to linearly weight the channels of the feature map together to obtain the heat map, as shown in Equation 2 below:

$$L_{Grad-CAM}^c = RELU(\sum_k a_k^c A^k) \qquad \text{(Equation 2)}$$

In Equation 2, Grad-CAM adds a linear weighting (Relu function) operation to the fused heat map, retaining only the regions that have a positive effect on category c, i.e. the image of the region of interest for the corresponding classification result output by the neural network model is obtained. An activation map is obtained by superimposing the heat map on the sample analysis image. In this way, the activation map can more intuitively show the importance of different regions in the sample analysis image for the identification result of the target feature, such that not only can the neural network model be fully utilized to identify and analyze the sample analysis image to compensate for the bias caused by individual differences among doctors, but also the neural network model can be fully utilized to realize more image features that are indistinguishable to the human eye to improve identification accuracy and identification precision. More importantly, it is possible to obtain the basis for the identification result of the target feature obtained by the neural network model based on the sample analysis image, and present the basis in a visual manner in the auxiliary diagnosis report.

In some embodiments, before the S06: based on the identification result of the target feature, performing a search in a sample feature library containing sample features corresponding to historical diagnosis cases to determine similar sample feature information corresponding to the identification result of the target feature, the auxiliary diagnosis method further includes: constructing a sample feature library corresponding to an identification of the target feature based on the identification result of the target feature of the to-be-tested object. Specifically, the step of constructing a sample image library based on the identification result of the target feature of the to-be-tested object includes: constructing the sample feature library corresponding to the identification result of the target feature by combining sample feature information corresponding to the target feature of each different tested object, i.e., the identification results of different target features correspond to different types of sample feature libraries. In addition, each of the sample feature libraries corresponding to different target feature types further includes a corresponding sample image library and a microscopic image library.

Referring to FIG. 6, in some embodiments, before the S06: based on the identification result of the target feature, performing a search in a sample feature library containing sample features corresponding to historical diagnosis cases to determine similar sample feature information corresponding to the identification result of the target feature, the auxiliary diagnosis method further includes S05: classifying each test object according to clinical information data of the test object, and establishing the sample feature library corresponding to each object type according to the object type and the historical diagnosis cases.

The classifying the test objects according to their clinical information data may mean classifying them according to one of: their gender, age, past medical history, and the diagnosis result categories, for example, classifying male test objects as one category and female test objects as another; or classifying test objects according to their age as adults or children; or classifying test objects with the same past medical history as one category. The object type refers to the type based on which the objects are classified. According to the object type and the historical diagnosis cases, the sample feature library corresponding to each object type is established, and each sample feature library corresponding to one object type may further include a corresponding sample image library and a microscopic image library. Specifically, sample images in the historical diagnosis cases corresponding to each tested object of the same object type are constructed into a sample image library corresponding to the object type, that is, the sample images in the historical diagnosis cases corresponding to the tested objects of different object types belong to different types of sample image libraries. Then, the abnormal sample images in the sample image library of a corresponding type are associated with the microscopic images to construct a microscopic image library of the corresponding type.

Continuing to refer to FIG. 6, the S061: according to the sample analysis image carrying the abnormality marker of the target feature, searching and determining a similar sample image from a sample image library containing sample analysis images corresponding to the historical diagnosis cases, includes: according to the clinical information data of the to-be-tested object, determining a target object type of the to-be-tested object; and according to the sample analysis image carrying the abnormality marker of the target feature, searching and determining the similar sample image from the sample image library corresponding to the target object type. That is, first, the object type to which the to-be-tested object belongs is determined based on the object type according to which the to-be-tested object is classified, the sample image library consistent with the object type of the to-be-tested object is determined from the constructed sample image libraries of different object types, and then from the sample image library, similar sample images that also indicate an abnormality of the current target feature are matched based on the identification result indicating the abnormality of the current target feature.

Further, the S062: according to the abnormality warning information, searching and determining a similar microscopic image from a microscopic image library containing microscopic images corresponding to the historical diagnosis cases, includes: according to the abnormality warning information, searching and determining the similar microscopic image from the microscopic image library corresponding to the target object type.

The identification result of the target feature of the to-be-tested object includes first identification information indicating an abnormality of the target feature, correspondingly, the similar sample image matching the identification result of the target feature includes second identification information indicating an abnormality of the target feature in the similar sample image, and the similar microscopic image includes third identification information characterizing the abnormality degree of the target feature. Each identification information may be a graphical identification information and/or a textual identification information. In an optional specific example, the identification information is a marking frame.

In some embodiments, the sample feature library further includes at least one piece of the following sample feature information: a device status reference feature that characterizes whether the status of a testing device is abnormal, an abnormal sample reference feature that characterizes whether the quality of the biological sample is abnormal, and an identification result reference feature that characterizes whether the identification result of the target feature is abnormal.

Further still, the S06: based on the identification result of the target feature, performing a search in a sample feature library containing sample features corresponding to historical diagnosis cases to determine similar sample feature information corresponding to the identification result of the target feature, further includes: according to the identification result of the target feature, determining a device status reference feature, an abnormal sample reference feature, and/or an identification result reference feature corresponding to the identification result of the target feature. The abnormal sample reference feature may also be referred to as a sample quality reference feature. In some embodiments, the sample feature library includes the device status reference feature, the abnormal sample reference feature, and the identification result reference feature, and the similar sample feature information obtained in the S06 includes the device status reference feature, the abnormal sample reference feature, and the identification result reference feature that match the identification result of the target feature. Based on the device status reference feature, abnormal sample reference feature, and identification result reference feature output in the auxiliary diagnosis report, it can be determined whether the testing device for the to-be-tested object is in normal condition, whether the quality of the biological sample used for testing the to-be-tested object is normal, and whether there are accidental external causes such as environmental or equipment interference in the identification result of the target feature, so as to provide further explanatory information on the accuracy of the subsequent auxiliary diagnosis results.

Optionally, the similar sample feature information corresponding to the identification result of the target feature includes a device status reference feature. During the determination of the auxiliary diagnosis result, when it is determined that the device status is abnormal according to the device status reference feature, the diagnosis basis corresponding to the current auxiliary diagnosis result in the auxiliary diagnosis report includes the sample analysis image indicating an abnormality of the target feature in the identification result of the target feature, and the device status reference feature corresponding to the similar sample feature information that characterizes whether the device status is abnormal, while the similar sample image may be omitted.

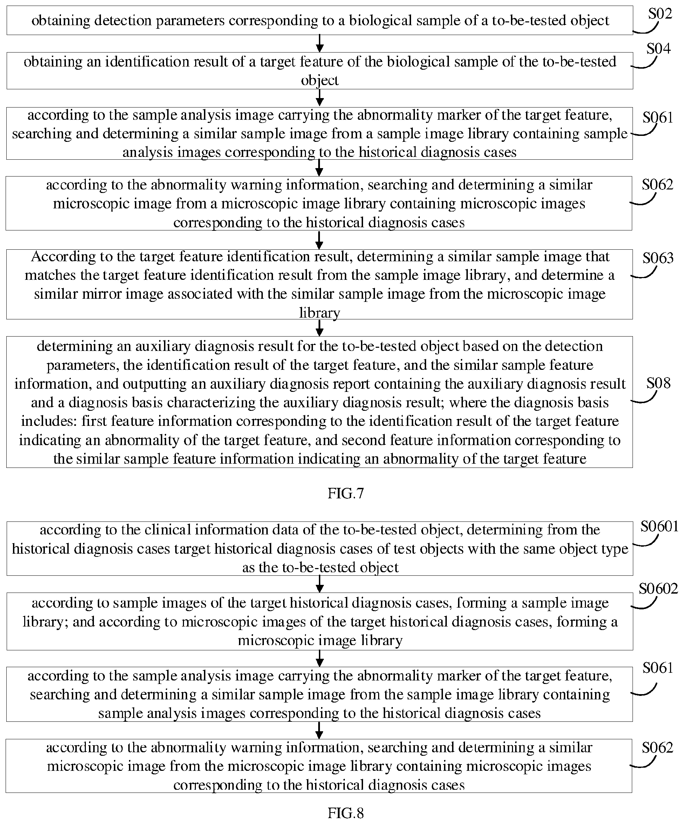

Referring to FIG. 7, in some embodiments, in the S062, the searching and determining the similar microscopic image from the microscopic image library corresponding to the target object type further includes: searching and determining the matching similar microscopic image from the microscopic image library corresponding to the target object type that matches a respective preset abnormality level. The similar microscopic image includes corresponding microscopic images of tested objects for different abnormality levels regarding the target feature. The different abnormality levels may be Normal, Mild, Moderate, and Severe. Further, when the target feature is a target feature most relevant to the diagnosis result, the corresponding similar microscopic image includes similar microscopic images that match the different abnormality levels.

In some embodiments, in the S061, the searching and determining the similar sample image from the sample image library corresponding to the target object type further includes: searching and determining a similar abnormal sample image with the highest similarity and a similar normal sample image with the highest similarity from the sample image library corresponding to the target object type. The similarity between each sample image in the sample image library corresponding to the target object type and the target identification result is obtained based on an image similarity algorithm, and an abnormal sample image corresponding to the abnormal target feature with the highest similarity is obtained, as well as a normal sample image corresponding to the normal target feature most similar to the target identification result. In some embodiments, the target feature is a target feature that contributes the most to the subsequent auxiliary diagnosis result of the to-be-tested object. The normal sample image can be applied by the user to compare with the abnormal sample image in the auxiliary diagnosis report. The greater the difference between the abnormal sample image and the normal sample image, the greater the abnormality degree of the target feature in the abnormal sample image.

Referring to FIG. 8, in some embodiments, before the S061 and S062, the S06: based on the identification result of the target feature, performing a search in a sample feature library containing sample features corresponding to historical diagnosis cases to determine similar sample feature information corresponding to the identification result of the target feature, further includes operations at blocks S0601 and S0602, as described in detail below.

At block S0601: according to the clinical information data of the to-be-tested object, determining from the historical diagnosis cases target historical diagnosis cases of test objects with the same object type as the to-be-tested object.

At block S0602: according to sample images of the target historical diagnosis cases, forming the sample image library; and according to microscopic images of the target historical diagnosis cases, forming the microscopic image library.

Further, the S061 includes: according to the sample analysis image carrying the abnormality marker of the target feature in the identification result of the target feature, searching from the sample image library corresponding to the object type of the to-be-tested object to determine the similar sample image that matches the sample analysis image. The S062 includes: according to the abnormality warning information, which characterizes the abnormality of the target feature, in the identification result of the target feature, searching from the microscopic image library corresponding to the object type of the to-be-tested object to determine the similar microscopic image that matches the abnormality warning information.

In the embodiments of the auxiliary diagnosis method shown in FIG. 8, the object type to which the to-be-tested object belongs may first be determined according to the clinical information data of the to-be-tested object, and then the historical diagnosis cases corresponding to the test objects with the same object type as the to-be-tested object are determined as the target historical diagnosis cases. The sample analysis images and the microscopic images contained in the target historical diagnosis cases are then constructed into the sample image library and the microscopic image library, respectively. Then, similar sample images and similar microscopic images are matched from the sample image library based on the identification result of the target feature. Since the sample image library corresponds to the object type of the to-be-tested object, the similar sample images and microscopic images obtained from it are more comparable to the sample image of the to-be-tested object carrying the abnormality marker.

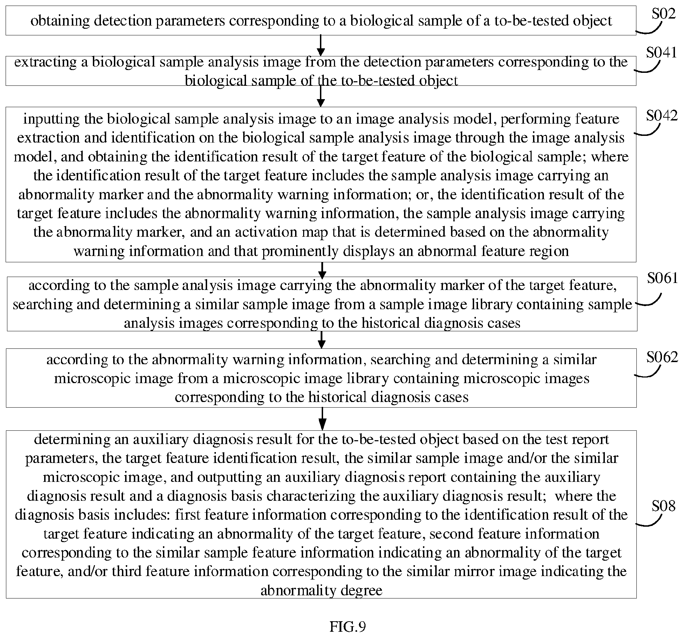

Referring to FIG. 9, in some embodiments, the S04: obtaining an identification result of a target feature of the biological sample of the to-be-tested object includes:

At block S041: extracting a biological sample analysis image from the detection parameters corresponding to the biological sample of the to-be-tested object.

From the test report parameters, pulse signals corresponding to the parameters of each testing channel of the sample analyzer can be obtained. A corresponding cell histogram is formed based on the pulse features of each channel, such as the peak height, leading peak width, trailing peak width, and half-peak width of the pulse signal, e.g., histograms of RBC, PLT, and WBC formed based on the peak height of the pulse signal. In addition, two-dimensional, three-dimensional, or even multi-dimensional scatter plots composed of features values of pulse signals in several dimensions, and two-dimensional scatter plot projections formed by three-dimensional and high-dimensional scatter data can be generated.

At block S042: inputting the biological sample analysis image to an image analysis model, performing feature extraction and identification on the biological sample analysis image through the image analysis model, and obtaining the identification result of the target feature of the biological sample; where the identification result of the target feature includes the sample analysis image carrying an abnormality marker and the abnormality warning information; or, the identification result of the target feature includes the abnormality warning information, the sample analysis image carrying the abnormality marker, and an activation map that is determined based on the abnormality warning information and that prominently displays an abnormal feature region.

Feature extraction is performed on the biological sample analysis image through the image analysis model to output the identification result of the target feature carried by the biological sample analysis image; the biological sample analysis image may include at least one of the following: blood cell histogram, blood cell scatter plot, pulse signal waveform diagram, pulse signal feature map, thromboelastogram, and biochemical or immune response curve. Feature extraction and identification is performed on the target feature in the biological sample analysis image through the image analysis model to obtain the abnormality warning information indicating whether the target feature is abnormal.

In some embodiments, the image analysis model is a deep learning-based model (AI model) or a conventional algorithm model. The performing a feature extraction on biological sample analysis data using the deep learning model or conventional algorithm model, and outputting the identification result of the target feature carried in the biological sample analysis data, include: performing a feature extraction on the biological sample analysis data using the deep learning model based on image classification, or, performing a feature extraction on the biological sample analysis data using the conventional algorithm model constructed based on image morphology algorithm, image classification algorithm, clustering algorithm, or threshold segmentation algorithm; and performing a classification according to results of the feature extraction, and outputting the identification result of the target feature carried in the biological sample analysis data.

In some embodiments, in the S042, the obtained identification result of the target feature further includes an activation map that is determined based on the abnormality warning information and that prominently displays an abnormal feature region.

The activation map may be a CAM category activation map. Taking the image analysis model being a classification neural network model as an example, it may include a pooling layer, and may be adopted with visual feature visualization algorithms such as Guided-Backpropagation, Grad-CAM, Score-CAM, and Group-CAM. The image analysis model may utilize a spatial correspondence between the feature map output by the last convolutional layer and the original image to mark the image region that determines a corresponding classification label (i.e., the abnormality warning information) as the region of greater interest of the neural network model on the original image, and output it synchronously with the classification label classification label. In addition, in some embodiments, the image analysis model may be a target detection neural network model.

Continuing to refer to FIG. 9, in some embodiments, before the S042: inputting the biological sample analysis image to an image analysis model, performing feature extraction and identification on the biological sample analysis image through the image analysis model, and obtaining the identification result of the target feature of the biological sample, the auxiliary diagnosis method further includes: training an initial neural network model using a sample image training set carrying a target feature label to obtain a trained image analysis model; where the target feature label includes: Normal Target Feature, Abnormal Target Feature and its abnormal location identification.

Further, continuing to refer to FIG. 9, the S042: inputting the biological sample analysis image to an image analysis model, performing feature extraction and identification on the biological sample analysis image through the image analysis model, and obtaining the identification result of the target feature of the biological sample, includes: inputting the biological sample analysis image into the image analysis model, performing the feature extraction and identification on the biological sample analysis image through the image analysis model, obtaining an identification result of the target feature of the biological sample indicating that the target feature of the biological sample is normal, or obtaining an identification result of the target feature indicating that the target feature of the biological sample is abnormal together with an abnormal location identification. The identification result of the target feature includes the identification result of the normal target feature and the identification result of the abnormal target feature, and the identification result of the abnormal target feature further includes the abnormality warning information, the sample analysis image of a target feature region marked with an abnormality, and an activation map displaying an abnormal target feature region.

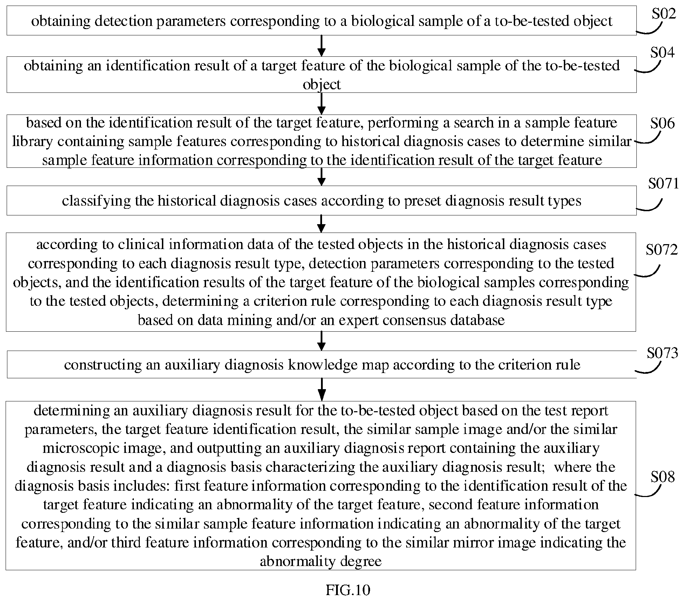

Referring to FIG. 10, in some embodiments, before the S08, the method further includes S07: constructing an auxiliary diagnosis knowledge map according to clinical information data and test report parameters of the historical diagnosis cases of the tested objects, and identification results of the target feature corresponding to the tested objects, i.e., the auxiliary diagnosis apparatus of the embodiments of the present disclosure performs the S08 step to determine the knowledge map of the auxiliary diagnosis category. Specifically, S07 includes operations at blocks S071, S072, and S073.

At block S071: classifying the historical diagnosis cases according to preset diagnosis result types.

The setting of the diagnosis result type refers to a preset rule for classifying the diagnosis results corresponding to the historical diagnosis cases according to the diagnosis result type. For example, different disease types may be set to correspond to different diagnosis result types; or, different disease types may be grouped first, and the diagnosis results of each disease type in each group belong to one type.

At block S072: according to clinical information data of the tested objects in the historical diagnosis cases corresponding to each diagnosis result type, detection parameters corresponding to the tested objects, and the identification results of the target feature of the biological samples corresponding to the tested objects, determining a criterion rule corresponding to each diagnosis result type based on data mining and/or an expert consensus database.

The data mining refers to the mining of the criterion rule corresponding to each diagnosis result type from the clinical information data, test report parameters, and identification results of the target feature corresponding to each diagnosis result type based on an AI data mining model, i.e., obtaining a respective clinical determination condition corresponding to the disease type in each diagnosis result. The expert consensus database refers to a library constructed based on the combination of expert knowledge, clinical guidelines, authoritative textbooks, and other prior expert consensus. The criterion rule is to determine a condition that needs to be met to determine the diagnosis result type of the test object.

At block S073: constructing an auxiliary diagnosis knowledge map according to the criterion rule.

In some embodiments, the criterion rules corresponding to each diagnosis result type in different testing modes are determined based on data mining and/or an expert consensus database. Different testing modes refer to the testing modes of the sample analyzer to analyze the biological sample of the test object. Taking a blood cell analyzer to analyze a blood sample as an example, the testing modes of the blood cell analyzer include a CD testing mode and a CDR testing mode. Among them, the CD detection mode is configured to obtain a complete blood count and classification (CBC+DIFF), and the CDR detection mode is configured to obtain a complete blood count and classification and test reticulocyte detection (CBC+DIFF+RET). The test report parameters include reporting parameters obtained in one testing mode of the blood cell analyzer or reporting parameters obtained in multiple testing modes.

Different disease diagnosis conditions require different analyses of the sample analyzer. For example, for disease A, the diagnosis condition requires that the sample analyzer test the biological sample using the complete blood-cell count (CBC) and five-part differential white blood cell count (i.e., the DIFF channel); therefore, the sample analyzer needs to test the biological sample in CD testing mode. For disease B, the diagnosis condition requires that the sample analyzer's detection of the biological sample include complete blood-cell count (CBC), differential white blood cell count (DIFF channel), and reticulocyte count (RET channel); therefore, the sample analyzer needs to test the biological sample in CDR testing mode. Therefore, regarding the step of determining a criterion rule corresponding to each diagnosis result type based on data mining and/or an expert consensus database, according to clinical information data of the tested objects in the historical diagnosis cases corresponding to each diagnosis result type, detection parameters corresponding to the tested objects, and the identification results of the target feature of the biological samples corresponding to the tested objects, testing mode information of the sample analyzer corresponding to the biological samples of the historical diagnosis cases may be obtained. The historical diagnosis cases may be classified according to the testing mode information to conclude the historical diagnosis cases under different testing modes. The corresponding criterion rule for each diagnosis result type under different testing modes may thus be determined according to the testing report parameters of the historical diagnosis cases under different testing modes and the identification results of the target feature of the biological samples corresponding to the tested objects, based on data mining and/or expert consensus database.

Combining the clinical information data, the test report parameters, and the identification result of the target feature of the to-be-tested object with the criterion rules under different testing modes can obtain the auxiliary diagnosis results and basis of the to-be-tested object under different testing modes. In this way, the auxiliary diagnosis apparatus is further configured to combine the clinical information data, the test report parameters, and the identification result of the target feature of the to-be-tested object with the corresponding criterion rules in different testing modes, output the auxiliary diagnosis report including the auxiliary diagnosis results for the to-be-tested object in different testing modes and the diagnosis basis that characterizes the auxiliary diagnosis results, such that the user can easily compare and decide whether to perform further testing on the to-be-tested object.

In some embodiments, the S072: determining a criterion rule corresponding to each diagnosis result type based on data mining and/or an expert consensus database, according to clinical information data of the tested objects in the historical diagnosis cases corresponding to each diagnosis result type, detection parameters corresponding to the tested objects, and the identification results of the target feature of the biological samples corresponding to the tested objects, is specifically implemented by: according to the clinical information data of the tested objects in the historical diagnosis cases corresponding to each diagnosis result type, the detection parameters corresponding to the tested objects, and the identification results of the target feature of the biological samples corresponding to the tested objects, determining the criterion rule corresponding to each diagnosis result type based on the data mining.

In some embodiments, the S073: constructing an auxiliary diagnosis knowledge map according to the criterion rule, further includes:

parsing the criterion rule to form a key-value pair for the criterion rule, where the key is the diagnosis result type and the value is a decision condition set corresponding to the diagnosis result type; and

forming a key-value pair database according to the key-value pair corresponding to the criterion rule for each diagnosis result type, and constructing the auxiliary diagnosis knowledge map according to the key-value pair database.

After obtaining the criterion rules, before entering them into the auxiliary diagnosis knowledge map, they can be converted into a dictionary format, i.e., json format, for easier reading. The dictionary format is in the form of {"key": "value"}. The criterion rule is parsed to extract the diagnosis result type in the criterion rule, such as the potential risk of disease A or the potential risk of disease B, and the determination condition in the criterion rule is obtained, such as "WBC$\geq$18$\times$10$^9$/L, PLT<90$\times$10$^9$/L, Blast: medium, IG: high", or "PLT<90$\times$10$^9$/L, HGB<90g/L, IG high, RET< 15 10$^9$/L" etc., then the key-value pair for the diagnosis result type of disease A is {"disease A": "WBC>=18 10^9/L, PLT<90 10^9/L, Blast: medium, IG: high"}, and the key-value pair for the diagnosis result type of disease B is {"Disease B": "PLT<90 10^9/L, HGB<90g/L, IG high, RET<15 10^9/L"}.

In some embodiments, before inputting the clinical information data, the test report parameters, the identification results of the target feature, the similar sample images, and the similar microscopic images of the to-be-tested object into the auxiliary diagnosis knowledge map, the criterion rule corresponding to the to-be-tested object is determined based on the clinical information data, the test report parameters, and the identification results of the target feature of the to-be-tested object, and test sample input data of the to-be-tested object is formed according to the corresponding criterion rule and input into the auxiliary diagnosis knowledge map. For easy reading, the test sample input data is converted into the dictionary format first, for example: {"sample 1": "WBC 5.6 10^9/L, PLT 80 10^9/L, HGB 70g/L, Blast negative, IG high, RET 10 10^9/L"}.

Continue to refer to FIG. 10, in some embodiments, the S08: determining an auxiliary diagnosis result for the to-be-tested object based on the detection parameters, the identification result of the target feature, and the similar sample feature information, and outputting an auxiliary diagnosis report containing the auxiliary diagnosis result and a diagnosis basis characterizing the auxiliary diagnosis result, includes:

constructing a first determination condition according to the clinical information data of the to-be-tested object;
constructing a second determination condition according to the test report parameters of the to-be-tested object;
constructing a third determination condition according to the identification result of the target feature of the biological sample of the to-be-tested object; and

inputting the first determination condition, the second determination condition, and the third determination condition into the auxiliary diagnosis knowledge map in parallel, and determining, through the auxiliary diagnosis knowledge map, the auxiliary diagnosis result of the to-be-tested object according to a compliance of the first determination condition, the second determination condition, and the third determination condition with the criterion rule of each diagnosis result type. The auxiliary diagnosis apparatus may, according to the auxiliary diagnosis result and the similar sample image and/or similar microscopic image, generate and output the auxiliary diagnosis report containing the auxiliary diagnosis result and the diagnosis basis representing the auxiliary diagnosis result according to a set report template type.

Specifically, the constructing a first determination condition according to the clinical information data of the to-be-tested object, includes: analyzing the clinical information data of the to-be-tested object, forming the clinical information data into a first sequence whose elements are the data types and their corresponding values respectively, and taking the first sequence as the first determination condition. For example, the first sequence is {"sample 1": "gender: male, age: 30, past medical history: hypertension"}. The constructing a second determination condition according to the test report parameters of the to-be-tested object, includes: parsing the test report parameters of the to-be-tested object, forming the test report parameters into a second sequence whose elements are the data types and their corresponding values respectively, and taking the second sequence as the second determination condition. For example, the second sequence is {"sample 1": "WBC 5.6 10^9/L, PLT 80 10^9/L, HGB 70g/L"}. The constructing a third determination condition according to the identification result of the target feature of the biological sample of the to-be-tested object, includes: analyzing the identification result of the target feature of the biological sample of the to-be-tested object, forming the identification result of the target feature into a third sequence whose elements are the data types and their corresponding values respectively, and taking the third sequence as the third determination condition. For example, the third sequence is { "sample 1": "Blast absent, IG high"}.

In some embodiments, the first determination condition is data after a dictionary conversion of the clinical information data of the to-be-tested object, the second determination condition is data after a dictionary conversion of the test report parameters of the to-be-tested object, and the third determination condition is data after a dictionary conversion of the identification result of the target feature of the to-be-tested object.

The compliance of the first determination condition, the second determination condition, and the third determination condition with the criterion rule of each diagnosis result type refers to the matching degree of each of the first determination condition, the second determination condition, and the third determination condition with the corresponding determination condition in the criterion rule. The higher the matching degree, the higher the possibility that the auxiliary diagnosis result type of the to-be-tested object will be consistent with the diagnosis type result corresponding to the criterion rule.

In some embodiments, the S08: determining an auxiliary diagnosis result for the to-be-tested object based on the detection parameters, the identification result of the target feature, and the similar sample feature information, and outputting an auxiliary diagnosis report containing the auxiliary diagnosis result and a diagnosis basis characterizing the auxiliary diagnosis result, specifically includes operations at blocks S081 and S082, as described below.

At block S081: determining the auxiliary diagnosis result of the to-be-tested object according to the clinical information data, the detection parameters, the identification result of the target feature, and the similar sample feature information of the to-be-tested object.

The clinical information data, the detection parameters, the identification result of the target feature, and the similar sample feature information of the to-be-tested object are input into the auxiliary diagnosis knowledge map, and the auxiliary diagnosis result of the to-be-tested object is obtained based on the knowledge map. Specifically, the auxiliary diagnosis result is determined by the compliance of the data input into the auxiliary diagnosis knowledge map and the criterion rule corresponding to the corresponding diagnosis result type in the knowledge map.

At block S082: according to a result type of the auxiliary diagnosis result, outputting the auxiliary diagnosis report matching the result type; where the first identification information, indicating an abnormality of the target feature, in the identification result of the target feature refers to a sample analysis image carrying an abnormality marker of the target feature; the second identification information, indicating an abnormality of the target feature, in the similar sample feature information includes a similar sample image obtained from the sample feature library carrying an abnormality marker of the target feature, and an activation map that is obtained from the sample feature library according to the abnormality warning information and that prominently displays an abnormal feature region; the third identification information indicating the abnormality degree of the target feature refers to a similar microscopic image obtained from the sample feature library carrying information indicative of the abnormality degree of the target feature. In some embodiments, the auxiliary diagnosis report includes a sample analysis image that carries an abnormality marker of the target feature, a similar sample image that carries an abnormality marker of the target feature, an activation map that prominently displays an abnormal feature region, and a similar microscopic image that characterizes different abnormality degrees. The target feature is a feature that is most relevant to the auxiliary diagnosis result. The similar microscopic images with different abnormality levels include a reference microscopic image indicating that the target feature is normal and microscopic images indicating that the target feature is with a different abnormality degree.

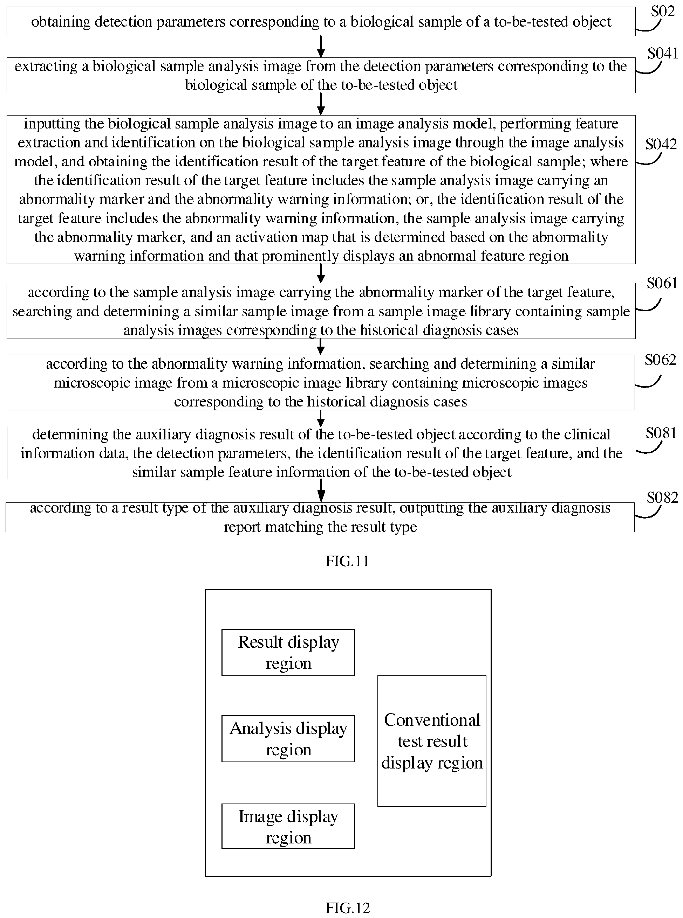

In some embodiments, referring to FIG. 12, the auxiliary diagnosis report template includes a result display region, an analysis display region, and an image display region, and further includes a conventional test result display region. The result display region is configured to display the auxiliary diagnosis result and further display a diagnosis recommendation obtained based on the auxiliary diagnosis result. The type of auxiliary diagnosis result includes: a first result type with an explicit risk result and a second result type with an implicit risk result. The explicit risk result refers to an auxiliary diagnosis type where the disease type that the to-be-tested object is likely to suffer from can be clearly obtained based on the information of the to-be-tested object entered into the auxiliary knowledge map; whereas the implicit risk result refers to a diagnosis result type where the disease type that the object suffers from cannot be clearly determined only based on the auxiliary diagnosis knowledge map, but only the abnormality warning information corresponding to the target feature in the biological sample and the clinical recommendations made based on the abnormality warning information can be determined. The result display regions corresponding to different types of auxiliary diagnosis results display different content, which will be described in detail below. The analysis display region mainly displays the diagnosis basis of the numerical and textual descriptions corresponding to the auxiliary diagnosis result, including the detection parameter information related to the auxiliary diagnosis result and the abnormality warning information in the identification result of the target feature. The image display region is configured to display the sample analysis image corresponding to the target feature most related to the auxiliary diagnosis result, and the activation map, the similar sample image, and the similar microscopic image corresponding to the abnormality of the target feature. The similar microscopic image includes a normal reference microscopic image and microscopic images of different abnormality degrees. The conventional detection display region is configured to display a test result obtained based on a conventional algorithm, including report parameter information, abnormality warning information, and corresponding sample analysis image information.

Therefore, further, the S082: according to a result type of the auxiliary diagnosis result, outputting the auxiliary diagnosis report matching the result type, specifically includes: in response to the auxiliary diagnosis result being of the first result type, displaying, by the auxiliary diagnosis report, the explicit risk result through the result display region, displaying a key evidence that led to the explicit risk result and a subsequent processing recommendation through the analysis display region, and displaying the corresponding sample analysis image, similar sample image, activation map, and similar microscopic image through the image display region; in response to the auxiliary diagnosis result being of the second result type, displaying, by the auxiliary diagnosis report, the abnormality warning information in the identification result of the target feature through the result display region, displaying a maximum suspected risk corresponding to the abnormality warning information and a subsequent processing recommendation through the analysis display region, and displaying the sample analysis image, similar sample image, activation map, and similarity microscopic image through the image display region. In some embodiments, the diagnosis results and analysis results on the result display region and the analysis display region are both obtained based on AI. After the abnormality warning information is obtained, the maximum suspected risk corresponding to the abnormality warning information is determined based on a mapping relationship between the abnormality warning information and the potential risk of the corresponding existing disease type, that is, the disease type with potential risk that is most likely associated with the abnormality warning information is determined, and a corresponding diagnosis recommendation is determined based on the association result.

For example, assuming that it can be determined that the to-be-tested object has disease A based on the information entered into the knowledge map, the auxiliary diagnosis result will be of the first result type. Therefore, the result display region will display that the to-be-tested object has a high risk of having disease A and that a further clinical diagnosis is recommended; the analysis display region will display the basis for the high risk of the to-be-tested object having disease A, such as what determination conditions the detection parameters meet, and further display the diagnosis recommendation, such as recommending further testing in combination with the clinical; the image display region will display the sample analysis images, similar sample images, activation maps, and similar microscopic images of the target feature with the greatest relevance to disease A. Here, the greatest relevance means that the target feature best indicates the disease type suffered by the to-be-tested object. Furthermore, in some embodiments, the analysis display region further includes an indication of abnormal instrument states and abnormal sample states for the biological sample of the to-be-tested object.

For example, when it is not possible to determine the disease type suffered by the to-be-tested object based on the information entered into the knowledge map, but it can be determined that feature B is abnormal based on the identification result of the target feature, then the auxiliary diagnosis result is of the second result type. The auxiliary diagnosis report then displays the abnormality warning information for feature B through the corresponding result display region, and displays a diagnosis recommendation for further confirmation in combination with the clinical, where the abnormality warning information for different target features correspond to different diagnosis recommendations. The analysis display region displays the disease type that may be caused by the abnormal feature B, that is, the disease type that the to-be-tested object may suffer from due to the abnormal feature B, and further displays prompt information based on further clinical diagnosis. The image display region displays an interpretable image display of the abnormality of feature B, and the interpretable image includes an activation map corresponding to the abnormality of feature B, a sample analysis image annotated with the abnormality of feature B, and a normal reference microscopic image, a moderately abnormal microscopic image, and a severely abnormal microscopic image with the highest similarity to feature B.

In some embodiments, in the S08, the basis diagnosis further includes at least one of the following: a device status reference feature that characterizes whether the status of the detection apparatus is abnormal corresponding to the current auxiliary diagnosis result, an abnormal sample reference feature that characterizes whether the quality of the biological sample is abnormal, an identification result reference feature that characterizes whether the identification result of the target feature is abnormal. The analysis display region of the auxiliary diagnosis report further displays the device status reference feature, the abnormal sample reference feature, and/or the identification result reference feature corresponding to the current auxiliary diagnosis result. The device status reference feature may be used to determine whether the testing device for the to-be-tested object is normal, the abnormal sample reference feature may be used to determine whether the biological sample for testing the to-be-tested object is normal, and the identification result reference feature may be used to determine whether the identification result of the to-be-tested object is normal. When all the reference features are normal, it means that the auxiliary diagnosis result is valid; when any of them is abnormal, it means that the current auxiliary diagnosis result may be invalid.

In some embodiments, in the S08, the auxiliary diagnosis report further includes a corresponding conventional test result of the to-be-tested object and a diagnosis basis of the conventional test result; where the conventional test result includes abnormality warning information obtained according to a conventional test method, and the diagnosis basis of the conventional test result includes at least a sample analysis image of the biological sample of the to-be-tested object. The sample analysis image includes a histogram and a scatter plot corresponding to the biological sample, such as a blood cell histogram and a blood cell scatter plot.

In some embodiments, the auxiliary diagnosis report further includes a current test result displayed in comparison with the conventional test result, the current test result including an auxiliary diagnosis result, a description of the key basis for the auxiliary diagnosis result, and a subsequent processing recommendation. In the auxiliary diagnosis report, the conventional test result and the current test result may be displayed alternatively or both may be displayed.

In some embodiments, in the S08, the auxiliary diagnosis report containing the auxiliary diagnosis result and the diagnosis basis characterizing the auxiliary diagnosis result may be output based on a preset report template, or based on a report template selected from candidate report templates, or based on a user-configured report template or an uploaded report template. Different auxiliary diagnosis reports may be output according to report templates obtained in different ways, for the user to compare. The auxiliary diagnosis report contains the auxiliary diagnosis result and the diagnosis basis, and further includes some diagnosis and treatment recommendations.

In some embodiments, in the S08, the auxiliary diagnosis report containing the auxiliary diagnosis result and the diagnosis basis characterizing the auxiliary diagnosis result, includes an auxiliary diagnosis report including a textual description of the diagnosis basis; a sample analysis image, a similar sample image, a corresponding activation map, and a similar microscopic image, that are each marked with the target feature; and a numerical description. The sample image includes a sample analysis image of the to-be-tested object marked with the target feature, and a similar sample image from another tested object similar to the sample analysis image of the to-be-tested object. The microscopic image includes a similar microscopic image corresponding to abnormal target feature obtained from the microscopic image library. The similar sample image includes a similar sample image with normal target feature and a similar sample image with abnormal target feature. The similar microscopic image includes microscopic images of different abnormality levels and a normal reference microscopic image.

Taking a blood sample as an example, the target feature in the biological sample may be correlated with the diagnosis results of various historical diagnosis cases, such as a low platelet count and an increased white blood cell count with immature granulocytes. The diagnosis and treatment recommendation for a low platelet count may be to recommend relevant tests such as autoantibody tests and platelet-related immunoglobulin tests to confirm the diagnosis, while the diagnosis and treatment recommendation for an increased white blood cell count with immature granulocytes may be to recommend relevant tests such as bone marrow cytology tests if necessary. The auxiliary diagnosis report provides sample images of the to-be-tested object corresponding to the abnormal target feature of the biological sample and similar sample images, as well as microscopic images of the to-be-tested object corresponding to the abnormal target feature and similar microscopic images, and a textual description of the corresponding diagnosis and treatment recommendations, which makes the auxiliary diagnosis result more readable, facilitates the testing physician's efficient and accurate understanding of the specific cause of the abnormality in the current test result, and provides a basis for understanding the process of forming the conclusions in the auxiliary diagnosis report, thereby facilitating the testing physician's decision-making accordingly.

In some embodiments, when the identification result of the target feature is of a first type, the auxiliary diagnosis report includes at least two similar sample images whose similarity satisfies a preset condition, and the abnormality levels of the at least the two similar sample images are the same; when the identification result of the target feature is of a second type, the auxiliary diagnosis report includes a similar sample image with the highest similarity and at least one reference sample image, and the abnormality levels of the similar sample image and the reference sample image are different.

For example, the first type may refer to platelet aggregation. When the identification result of the target feature corresponding to the sample analysis image is platelet aggregation, multiple similar sample images with the highest similarity to the sample analysis image may be found in the sample analysis image library and displayed in the auxiliary diagnosis report. The second type is other abnormal feature information other than the first type. Optionally, the second type includes other abnormal information other than platelet aggregation. For example, the second type may include the presence of reactive lymphocytes, primitive cells, immature granulocytes, etc.

In the above embodiments, the sample analysis image library is set to be compatible with the identification results of target features of different types to match corresponding reference images. For some case types, there may be cases where the abnormality level is not distinguished, or there may not be enough historical diagnosis cases collected to distinguish between multiple abnormality levels, or there may be cases where doctors do not need to refer to multiple abnormality levels. Thus, the sample image library corresponding to the identification results of target features of some types contains images that do not distinguish between abnormality levels. For some case types, the sample image library containing the identification results of target features of corresponding categories to be considered for confirming the suspected case type is set to include reference images of different abnormality levels, such that multiple reference images of different abnormality levels can be matched in the sample analysis image library, which is conducive to improving the accuracy of the auxiliary diagnosis result and further effectively improves the reference value of the auxiliary diagnosis report for the doctor.

In some embodiments, the auxiliary diagnosis method further includes: determining a matched image set in the sample image library according to the category of the identification result of the target feature; determining a similar sample image whose similarity satisfies a preset condition based on a similarity between the sample analysis image and a reference image in the matched image set; and according to a mode category to which the similar sample image belongs, determining a reference sample image in the sample image library belonging to the mode category same as the similar sample image and whose abnormality level is different from that of the similar sample image.

In some embodiments, the auxiliary diagnosis report further includes: a reference sample image that matches the sample analysis image and has a different abnormality level from the similar sample image; the abnormality level of the similar sample image is one of severe, moderate, and mild, and the abnormality level of the reference sample image includes the other two of severe, moderate, and mild.

It should be noted that the similar sample image and the reference sample image can also be reference images carrying positioning markers. The positioning marker may be added in the following ways: the sample analysis images of the historical diagnosis cases used as the basis for establishing the sample image library may originally carry positioning markers; or, after a matching reference image is found in the sample analysis image library based on the currently obtained sample analysis image carrying a marker, a positioning marker can be added to the matching reference image accordingly based on the position of the positioning marker in the sample analysis image.

In the above embodiments, the reference sample image is further displayed in the auxiliary diagnosis report, where the similar sample image and the reference sample image together form a reference image combination with abnormality levels of severe, moderate and mild, such that the doctor can directly make a more reliable determination based on the reference images of different abnormality levels output by the auxiliary diagnosis report, and maximize the value of the process of completing the identification of the sample analysis image based on machine learning to obtain an auxiliary diagnosis conclusion, while ensuring the provision of reliable reference.

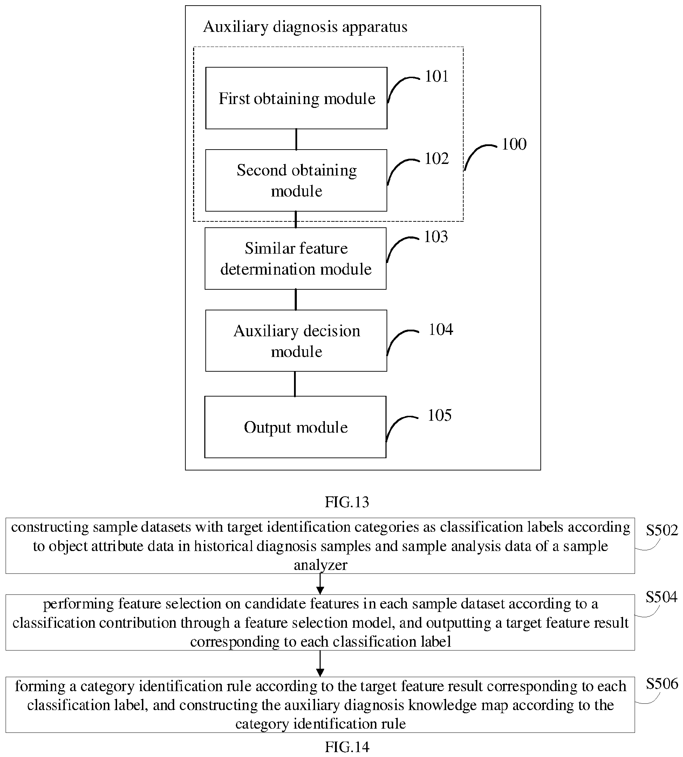

Referring to FIG. 13, in some embodiments, the present disclosure further provides an auxiliary diagnosis apparatus. The auxiliary diagnosis apparatus includes a first obtaining module 101, a second obtaining module 102, a similar feature determination module 103, and an auxiliary decision-making module 104. The first obtaining module 101 is configured to obtain detection parameters corresponding to a biological sample of a to-be-tested object; the second obtaining module 102 is configured to obtain an identification result of a target feature of the biological sample of the to-be-tested object; the similar feature determination module 103 is configured to search from a sample feature library containing sample features corresponding to historical diagnosis cases according to the identification result of the target feature, to determine similar sample feature information corresponding to the identification result of the target feature; the auxiliary decision-making module 104 is configured to determine an auxiliary diagnosis result for the to-be-tested object based on clinical information data, detection parameters, the identification result of the target feature, and the similar sample feature information of the to-be-tested object, and output an auxiliary diagnosis report containing the auxiliary diagnosis result and a diagnosis basis characterizing the auxiliary diagnosis result; where the diagnosis basis includes a sample analysis image characterizing an abnormality of the target feature in the identification result of the target feature and a similar sample image characterizing an abnormality of the target feature in the similar sample feature information.

It should be noted that the above-mentioned example provides an auxiliary diagnosis apparatus in the process of implementing an auxiliary diagnosis method, and only the above division of program modules is used as an example for explanation. In actual applications, the above processing may be assigned to different program modules as needed, that is, the internal structure of the apparatus may be divided into different program modules to complete all or part of the method steps described above. For example, the first obtaining module 101 and the second obtaining module 102 form an obtaining module 100; the auxiliary diagnosis apparatus includes an output module 105 for outputting the auxiliary diagnosis report containing the auxiliary diagnosis result and the diagnosis basis characterizing the auxiliary diagnosis result; etc. In addition, the auxiliary diagnosis apparatus provided in the above embodiments is of the same general idea as the embodiments of the auxiliary diagnosis method. Each component of the auxiliary diagnosis apparatus should be understood to be a functional module established corresponding to each step of the computer program for implementing the auxiliary diagnosis method. The specific implementation process is described in detail in the method embodiments and will not be repeated here.

In some embodiments, the present disclosure further provides a sample analyzer, including a sampling assembly, configured to collect and dispense a biological sample of a to-be-tested object, where the biological sample is a blood sample; a reaction assembly, configured to process the biological sample to form a to-be-tested solution; a drive assembly, configured to drive a liquid path between the sampling assembly and the reaction assembly; a detection component, configured to classify and count blood cells contained in the to-be-tested solution to obtain sample detection data comprising sample detection parameters and a sample analysis image; and the auxiliary diagnosis apparatus according to the present disclosure. The sample analyzer may be a stand-alone sample analyzer or a cascade sample analyzer.

In some embodiments, the present disclosure further provides another sample analysis system, the sample analysis system including the auxiliary diagnosis apparatus provided according to the present disclosure and a stand-alone sample analyzer or a cascade sample analyzer connected to the auxiliary diagnosis apparatus.

The cascade sample analyzer includes two or more sample analyzers cascaded together.

In some embodiments, as an alternative embodiment of the auxiliary diagnosis apparatus, the implementation of the auxiliary diagnosis method in the embodiments of the present disclosure may be deployed on a cloud server. The cloud server includes a processor and a memory, the memory storing a computer program executable by the processor, the computer program being executable by the processor according to the steps of the auxiliary diagnosis method of any embodiment of the present disclosure.

It should be noted that in the auxiliary diagnosis method described in the preceding embodiments, the implementation method of constructing the auxiliary diagnosis knowledge map in the S07 is not limited to the implementation method of constructing the knowledge map by determining the criterion rule based on data mining and/or an expert consensus database. The following describes the implementation method of an auxiliary diagnosis method, in which the construction method of the knowledge map is based on a technical idea of determining the classification contribution of features through a feature selection model.

The present disclosure further provides an apparatus for constructing an auxiliary diagnosis knowledge map. The apparatus for constructing the knowledge map refers to a computer program product, such as various application programs; it may refer to an auxiliary diagnosis apparatus loaded with the corresponding computer program product to realize the knowledge map construction function (i.e., the method for constructing an auxiliary diagnosis knowledge map described in any of the following embodiments of the present disclosure), such as various intelligent devices with storage and computing capabilities.

Referring to FIG. 14, in some embodiments, the method for constructing the auxiliary diagnosis knowledge map may be applied to the auxiliary diagnosis apparatus. The method includes, but is not limited to, operations at blocks S502, S504 and S506, which will be described below.

At block S502: constructing sample datasets with target identification categories as classification labels according to object attribute data in historical diagnosis samples and sample analysis data of a sample analyzer.

Each historical diagnosis sample refers to a historical diagnosis sample that is composed of object attribute data and sample analysis data corresponding to a historical test object in a historical diagnosis record. In the historical diagnosis sample, the corresponding object attribute data and feature information contained in the sample analysis data are taken as features in the historical diagnosis sample. In the embodiments of the method for constructing the auxiliary diagnosis knowledge map, the historical diagnosis sample is the same as the historical diagnosis case in other embodiments.

The sample analysis data refers to corresponding analysis data obtained by testing and analyzing biological information features in various types of test samples containing biological cell information or other biological information of the tested object. In an example, blood is treated as the test sample, biological information features corresponding to the blood include blood cell features. The blood cell features may be healthy features or unhealthy features presented in v sample corresponding to various types of cells, such as abnormal features in blood cells, cell types, cell quantity features, cell size features, cell composition ratio features, cell content features, nucleic acid content features, and so on. In order to achieve different test requirements, different target biological information features are to be tested when testing and analyzing the test samples. For blood samples, the target biological information features include one or more cell features. For example, for a PLT histogram in cell analysis data of the blood sample, the target cell features refer to quantitative distribution features of platelets in various sizes.

At block S504: performing feature selection on candidate features in each sample dataset according to a classification contribution through a feature selection model, and outputting a target feature result corresponding to each classification label.

Each historical diagnosis sample data in the sample dataset is input into the feature selection model. The feature selection model learns a mapping relationship between each feature and a corresponding classification label from the historical diagnosis sample data and calculates a contribution level of each feature in determining the classification. The classification contribution of the feature is a probability of determining the classification result as the corresponding classification label when the determining is performed based on the feature. As the probability is higher, the corresponding classification contribution is higher. Each candidate feature corresponds to one feature score. As the classification contribution of the candidate feature is higher, the corresponding feature score is higher, and vice versa. The target feature result is a feature identification result selected from the candidate features of each diagnosis historical diagnosis sample in the corresponding group of historical diagnosis samples, and a relevance between the feature identification result and the corresponding classification label meets a preset condition.

For example, the corresponding classification label is a disease A. A disease diagnosis category of the classification label of the sample dataset is having the disease A or being normal. The features of the sample dataset include the gender information, age information, and past medical history information in the object attribute data. The sample analysis data includes white blood cell count information, red blood cell count information, platelet count information, and other reported parameter information, as well as abnormality warning information obtained based on the sample analysis data, such as abnormality warning information about immature granulocytes.

The sample dataset is input into the feature selection model to train the feature selection model to learn the mapping relationship between each feature and the corresponding classification label. After the training is complete, the feature model ranks importance of each feature according to the classification contribution corresponding to each feature and selects a feature that meets the preset condition (such as selecting a feature having the classification contribution at a higher position of the rank) from a ranking result to form the target feature result. For example, for the disease A, the corresponding target feature result may be the white blood cell count information, the platelet count information, and the immature granulocyte warning information in the historical diagnosis sample. That is, it is determined, based on the white blood cell count information, the platelet count information, and the immature granulocyte warning information, whether the tested object has the disease A. Therefore, the target feature result is a clinical disease determination condition of the corresponding classification label. The feature selection model extracts the clinical disease determination condition corresponding to a disease type from the sample dataset having the historical diagnosis samples, and a doctor does not need to split up respective test data and combine the split test data with an experience of the doctor to determine the clinical disease determination condition.

At block S506: forming a category identification rule according to the target feature result corresponding to each classification label, and constructing the auxiliary diagnosis knowledge map according to the category identification rule.

The category identification rule is a rule corresponding to identifying a target identification category, i.e., a determination condition corresponding to determining the target identification category. When the target identification category is a disease diagnosis category, and the disease diagnosis category is the disease A in an example, the category identification rule is the clinical disease determination condition for the disease A. When a sample dataset taking the disease A as the classification label is constructed based on the object attribute data and the sample analysis data of the historical test object, the feature selection model is used to obtain the features result of a feature 1, a feature 2, and a feature 3, and the feature 1, the feature 2, and the feature 3 are the clinical disease determination condition for determining whether the tested object has the disease A. Therefore, in a subsequent auxiliary diagnosis process for the tested object, it is determined whether the tested object meets the feature 1, the feature 2, and the feature 3 based on the object attribute data and the sample analysis data of the tested object. When the tested object meets the feature 1, the feature 2, and the feature 3, the diagnosis result of the auxiliary diagnosis is that the tested object may have the disease A.

Each category identification rule includes: a corresponding target identification category, a corresponding target feature result, and a mapping relationship between the corresponding target identification category and the corresponding target feature result. In some embodiments, constructing the auxiliary diagnosis knowledge map based on the target category identification rule, includes: taking the target feature result and the corresponding target identification category to form an entity set in the auxiliary diagnosis knowledge map; and taking a relationship between the target identification category and the corresponding target feature result to form a relationship set in the auxiliary diagnosis knowledge map. The auxiliary diagnosis knowledge map is constructed based on the entity set and the relationship set.

As can be seen from the above, in the auxiliary diagnosis method provided by the present disclosure, the sample dataset having the disease diagnosis category as the classification label is constructed based on the object attribute data of the historical diagnosis cases and the analysis data of the tested samples. The feature selection model selects corresponding target features from the sample dataset according to the classification contribution of each feature. Further, the category identification rule is formed based on the target feature result corresponding to each classification label. The auxiliary diagnosis knowledge map is constructed based on the category identification rule. When constructing the auxiliary diagnosis knowledge map, there is no need to rely on any manual sorting method of the doctor to provide the disease determination condition, such that labor and time costs are effectively reduced.

In some embodiments, in the S506, after constructing the auxiliary diagnosis knowledge map based on the category identification rule, the method further includes: extracting entities and relationships from the category identification rule to construct the auxiliary diagnosis knowledge map. Before constructing the auxiliary diagnosis knowledge map based on the relationship between the classification label and the corresponding target feature result, the target feature result needs to be assessed to determine whether the target feature result is in consistent with an actual determination condition for the diagnosis category of the corresponding historical test object. When being consistent, the auxiliary diagnosis knowledge map is constructed based on the relationship between the classification label and the corresponding target feature result.

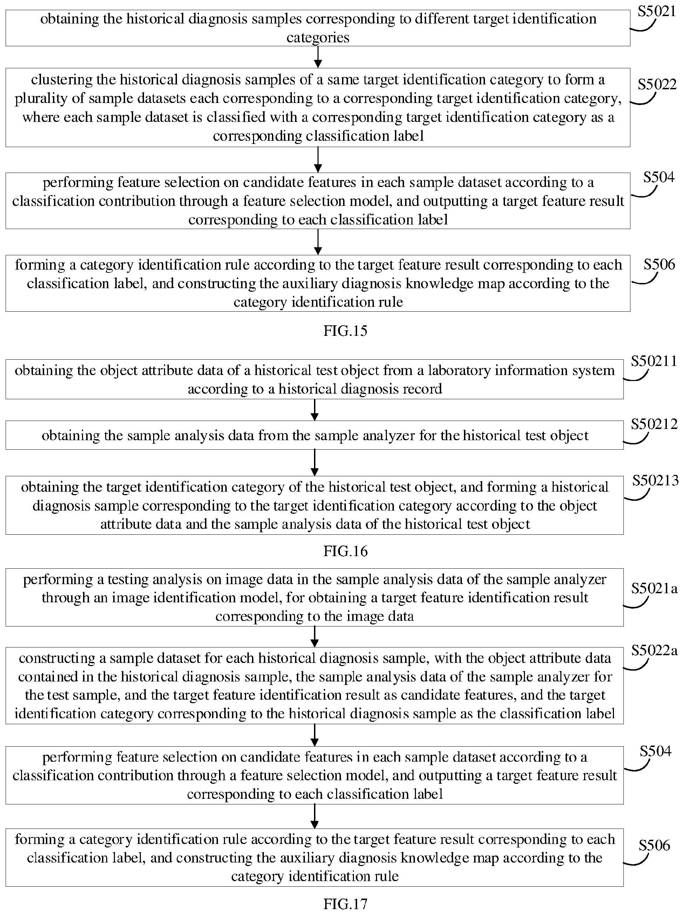

As shown in FIG. 15, in some embodiments, the S502: constructing sample datasets with target identification categories as classification labels according to object attribute data in historical diagnosis samples and sample analysis data of a sample analyzer, further includes operations at blocks S5021 and S5022, which will be described in detail below.

At block S5021: obtaining the historical diagnosis samples corresponding to different target identification categories. Each historical diagnosis sample includes object attribute data and sample analysis data of the same tested object. The object attribute data includes at least clinical information about the tested object. The clinical information includes information used to characterize an identity of the tested object and information used to determine reference parameters matching the tested object. The sample analysis data includes information about the test analysis result of the test sample of the tested object.

In the historical medical records corresponding to the historical test object, each medical record has a corresponding medical result. The medical result includes a diagnosis category of the disease of the tested object. However, in a case that the test result of the tested object cannot diagnose the disease of the tested object, it may be that the tested object does not have any disease, or it may be that an abnormality exists in the test sample or in the sample analyzer used for the test sample, and in this case, the diagnosis result is recorded as indicating that the sample or the device has abnormality. In some embodiments, the medical result further includes state category information of the test sample and/or state category information of the device. Therefore, the target identification category may further include a test sample state category and/or a device status category, in addition to the disease diagnosis category in the medical result. The device status category includes an abnormal state category of the sample analyzer, such as blocked holes. The test sample state category includes an abnormal state category of the test sample, such as red blood cell agglutination, platelet aggregation, or the test sample being an abnormal sample (such as a sample having abnormal primitive cells). In some embodiments, the target identification category includes a disease diagnosis category, and different disease diagnosis categories correspond to different clinical determination conditions. Therefore, in the present embodiment, the sample dataset includes historical diagnosis samples corresponding to different disease diagnosis categories. It should be noted here that in the present disclosure, different disease diagnosis categories include a normal type, i.e., the diagnosis result corresponding to the tested object is normal, and that is, the tested object is currently not suffering from the disease corresponding to the target classification label. The information that presents the identity of the tested object can be, such as, an ID number of the tested object in a historical diagnosis record library, i.e., the ID information. Each object attribute data of the tested object and each sample analysis data can be correlated to each other based on the ID information.

At block S5022: clustering the historical diagnosis samples of a same target identification category to form a plurality of sample datasets each corresponding to a corresponding target identification category, where each sample dataset is classified with a corresponding target identification category as a corresponding classification label.

Clustering the historical diagnosis samples means that attribute values of same features in all historical samples of the same target identification category and attribute values of target identification categories form rows of a label matrix; and categories of different features in all historical samples of the same target identification category and the target identification category form columns of the label matrix. In this way, the sample dataset corresponding to one target identification category is formed. Alternatively, clustering the historical diagnosis samples means that the attribute values of same features in all historical samples of the same target identification category and the attribute values of the target identification category form columns of the label matrix; and the categories of different features in all historical samples of the same target identification category and the target identification category form the rows of the label matrix. In this way, the sample dataset corresponding to one target identification category is formed.

As shown in FIG. 16, in some embodiments, the S5021: obtaining the historical diagnosis samples corresponding to different target identification categories, further includes operations at blocks S50211, S50212, and S50213, which will be described in detail below.

At block S50211: obtaining the object attribute data of a historical test object from a laboratory information system according to a historical diagnosis record.

Each historical test object (historical patient) has one corresponding historical diagnosis record after each visit. The historical diagnosis record includes all data related to the visit of the historical test object, such as the object attribute data and the sample analysis data of the historical test object. The historical diagnosis sample is a sample extracted from the historical diagnosis record corresponding to the historical diagnosis case and includes the object attribute data and the sample analysis data.

At block S50212: obtaining the sample analysis data from the sample analyzer for the historical test object.

The sample analyzer is configured to test and analyze the test sample of the tested object to obtain feature information of each biological information in the test sample. In an example, the test sample is the blood sample. A blood cell analyzer tests and analyzes the blood sample to obtain each feature information of blood cells. For example, the blood cell analyzer obtains reported parameter data and abnormal test data in the blood sample based on traditional algorithms. The reported parameter data includes feature information such as a count, a volume, and/or distribution of each type of cells in the blood sample, such as a white blood cell count, a red blood cell count, a platelet count, hematocrit, a mean size of red blood cells, a mean hemoglobin content of red blood cells, a mean hemoglobin concentration of red blood cells, distribution width of red blood cells, a standard deviation of the distribution width of red blood cells, and the platelet count.

In the S50211, the obtained object attribute data includes the ID information of the historical test object, and the historical test object having the same ID information is determined from the sample analyzer based on the ID information, and the sample analysis data of the historical test object having the same ID information is obtained.

At block S50213: obtaining the target identification category of the historical test object, and forming a historical diagnosis sample corresponding to the target identification category according to the object attribute data and the sample analysis data of the historical test object.

In some embodiments, the target identification category of each historical test object can be obtained from the historical diagnosis record, all features of the object attribute data and the sample analysis data of the same historical test object are combined according to the ID information of the historical test object to form the candidate features in the corresponding historical diagnosis sample. The candidate features are combined with the corresponding target identification category to form one historical diagnosis sample having the target identification category as the classification label.

In some embodiments, the S0212: obtaining the sample analysis data from the sample analyzer for the historical test object, includes: obtaining sample analysis data of a test sample of the same historical test object from multiple sample analyzers. The test sample of the historical test object includes a blood sample. Sample analysis data of the historical test object obtained from the sample analyzers form the corresponding historical diagnosis sample. In this way, the historical diagnosis sample includes more candidate features, such that accuracy of the feature selection model determining the clinical disease determination condition may be improved.

In some embodiments, the S021: obtaining the historical diagnosis samples corresponding to different target identification categories, further includes: obtaining a test sample state parameter corresponding to the historical diagnosis record from the laboratory information system based on the historical diagnosis record; forming the historical diagnosis sample corresponding to the target identification category based on the object attribute data, the sample analysis data, and the test sample state parameter of the same historical test object. The sample state parameter includes: a test sample state parameter, which includes the test sample state category, such as red blood cell agglutination, platelet aggregation, or the test sample being an abnormal sample (such as the sample having abnormal primitive cells). Therefore, in the present embodiment, the candidate features in the sample dataset having the target identification category as the classification label include the test sample state parameter, and that is, the test sample state parameter is a part of the candidate features. The target identification category further includes the test sample state category.

In some embodiments, the S021: obtaining the historical diagnosis samples corresponding to different target identification categories, further includes: obtaining a device status parameter corresponding to the historical diagnosis record from the sample analyzer; forming one historical diagnosis sample corresponding to the target identification category based on the object attribute data, the sample analysis data, and the device status parameter of the same historical diagnosis object. The device status parameter includes a device status category of the sample analyzer, such as an abnormal state of the sample analyzer (such as blocked holes). Therefore, in the present embodiment, the candidate features in the sample dataset having the target identification category as the classification label includes the device status parameter, i.e., the device status parameter is a part of the candidate features. The target identification category further includes the device status category.

In some embodiments, as shown in FIGS. 17, the S502: constructing sample datasets with target identification categories as classification labels according to object attribute data in historical diagnosis samples and sample analysis data of a sample analyzer, includes the following.

At block S5021a: performing a testing analysis on image data in the sample analysis data of the sample analyzer through an image identification model, for obtaining a target feature identification result corresponding to the image data.

The image identification model is an AI identification model, which is a neural network model based on deep learning. In some embodiments, the image data may be a histogram or scatter plot of blood cells. The image data is input into the image identification model, the image identification model is used to extract and identify features from the image data, and the target feature identification result corresponding to the image data is obtained. The target feature identification result includes abnormality warning data and a sample analysis image carrying abnormal annotations.

At block S5022a: constructing a sample dataset for each historical diagnosis sample, with the object attribute data contained in the historical diagnosis sample, the sample analysis data of the sample analyzer for the test sample, and the target feature identification result as candidate features, and the target identification category corresponding to the historical diagnosis sample as the classification label.

In the present embodiments, the candidate features include the object attribute data, the sample analysis data obtained by the sample analyzer, and the target feature identification result obtained based on the sample analysis data. In an example, the blood sample is the test sample. The AI identification model is used to perform feature extraction and identification from the sample analysis data obtained by the sample analyzer (such as the reported parameter data) to identify abnormal blood cell features in the test sample and to obtain warning data corresponding to the abnormal blood cell features, such as an IG abnormality warning or a Blast abnormality warning.

In some embodiments, the S502: constructing sample datasets with target identification categories as classification labels according to object attribute data in historical diagnosis samples and sample analysis data of a sample analyzer, includes: constructing the sample dataset taking the target identification category as the classification label based on the object attribute data contained in each historical diagnosis sample, the reported parameter data of the test sample by the sample analyzer, and the abnormal test data. That is, in the present embodiment, the sample analysis data further includes reported parameter information and an abnormal test result obtained by the sample analyzer according to traditional algorithms. The traditional algorithm includes, such as, image morphology algorithms, image classification algorithms, clustering algorithms or threshold segmentation algorithms. A traditional algorithm model constructed based on the above traditional algorithms constructed performs A traditional algorithm model on the sample analysis data to obtain abnormal test data that presents the test sample being abnormal. Therefore, in the present embodiment, the candidate features for the sample dataset include the object attribute data, the report parameter data, and the abnormal test data.

In addition, in some embodiments, the S502: constructing sample datasets with target identification categories as classification labels according to object attribute data in historical diagnosis samples and sample analysis data of a sample analyzer, includes: constructing the sample dataset having the target identification category as the classification label based on the image data from the sample analysis data of the sample analyzer, abnormality warning data obtained based on the AI identification model, abnormal test data obtained based on the sample analysis data by using the traditional algorithms; where the object attribute data contained in each historical diagnosis sample, the reported parameter data in the sample analysis data, the abnormality warning data, and the abnormal test data are used as the candidate features.

In some embodiments, the S504: performing feature selection on candidate features in each sample dataset according to a classification contribution through a feature selection model, and outputting a target feature result corresponding to each classification label, includes: ranking the candidate features in the sample dataset according to the classification contribution using a decision tree model; and outputting a target feature result corresponding to the classification label corresponding to the sample dataset according to a ranking result. Alternatively, in some embodiments, the S504: performing feature selection on candidate features in each sample dataset according to a classification contribution through a feature selection model, and outputting a target feature result corresponding to each classification label, includes: ranking the candidate features in the sample dataset according to the classification contribution using a random forest model; and outputting a target feature result corresponding to the classification label corresponding to the sample dataset according to a ranking result

The decision tree model or the random forest model learns the mapping relationship between each candidate feature and the corresponding classification label in the sample dataset; calculates the classification contribution of each candidate feature; and then ranks all candidate features from small to large or from large to small according to the classification contribution. After the ranking, the target feature result output is selected from the ranked candidate features according to the preset feature extraction conditions, where either a preset first or a preset last feature is selected as the target feature result to be output. In some embodiments, the target feature result is a feature combination formed by a preset number of features corresponding to top ranked features in terms of the classification contribution from all candidate features in the sample dataset.

In some embodiments, the historical diagnosis samples in the sample dataset are divided into three groups according to a preset ratio. One of the three groups is a training sample set; another one of the three groups is a verification sample set; and the rest one of the three groups is a test sample set. The training sample set is used to train the feature selection model to perform classification and feature selection, the validation sample set is used to verify classification and feature selection results of the feature selection model, and the test sample set is used to test classification and features selection performance of the feature selection model. Eventually, a trained feature selection model, which can classify based on input features and calculate the classification contribution of input features and rank and select features based on the classification contribution, is obtained. In some embodiments, the number of historical diagnosis samples in the training sample set, the number of historical diagnosis samples in the validation sample set, and the number of historical diagnosis samples in the test sample set is in the ratio of 8:1:1.

In some embodiments, the S506: forming a category identification rule according to the target feature result corresponding to each classification label, further includes: forming one category identification rule corresponding to the classification label based on the target feature result corresponding to each classification label, and based on the target feature, which has a rank position satisfying a preset condition, or based on correspondence between the target feature, which has a rank position satisfying a preset condition, and the classification label.

In some embodiments, a combination of candidate features having the classification contribution greater than a preset value is selected as the target feature result; or a combination of candidate features selected from a sequence of candidate features ranked based on classification contribution is selected as the target feature result when ranking positions thereof satisfy preset positions. Each set of target feature results is mapped to a corresponding classification label to form one diagnosis case corresponding to the classification label. In an example, the disease A is used as the classification label. When the target feature result output by the feature selection model is a combination of features of the white blood cell count, the platelet count, and the abnormal composition of immature granulocytes, a mapping relationship between the above combination of features and the disease A form one diagnosis case. The formed diagnosis case indicates that it can be determined that a corresponding tested object has the disease A when the clinical determination condition of white blood cells is that the white blood cell count, the platelet count, and the abnormal immature granulocytes all meet corresponding reference values.

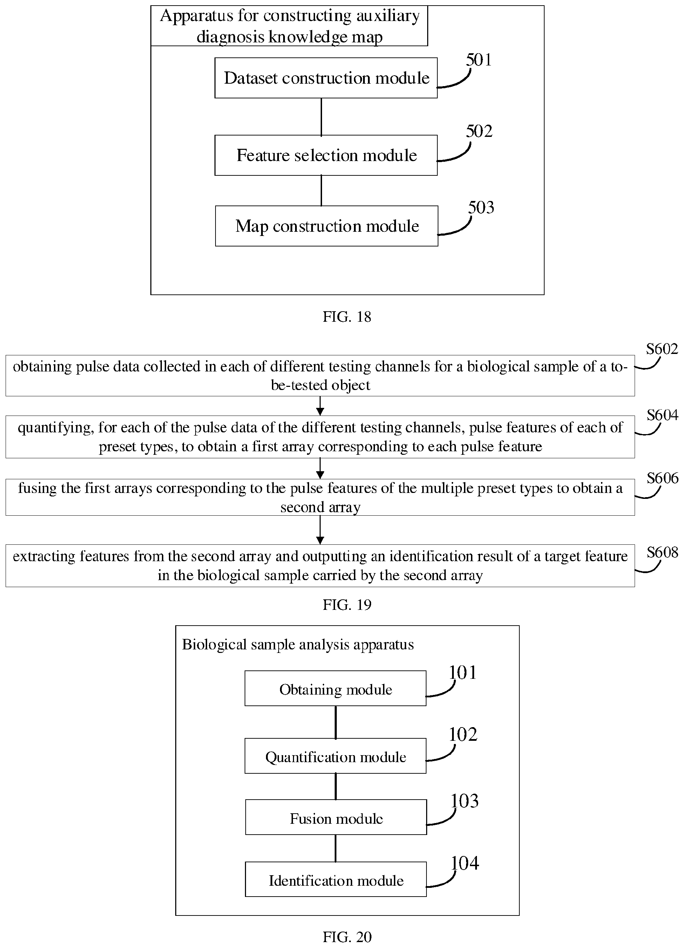

In another aspect of the present disclosure, referring to FIG. 18, an apparatus for constructing an auxiliary diagnosis knowledge map is further provided, including: a dataset construction module 501, a feature selection module 502, and a map construction module 503. The dataset construction module 501 is configured to construct sample datasets with target identification categories as classification labels according to object attribute data in historical diagnosis samples and sample analysis data of a sample analyzer. The feature selection module 502 is configured to perform feature selection on candidate features in each sample dataset according to a classification contribution through a feature selection model, and output a target feature result corresponding to each classification label. The map construction module 503 is configured to form a category identification rule according to the target feature result corresponding to each classification label, and construct the auxiliary diagnosis knowledge map according to the category identification rule.

It should be noted that: the construction apparatus for the auxiliary diagnosis knowledge map provided in the above embodiments is only exemplified by the above division of program modules in the process of implementing the construction method of the auxiliary diagnosis knowledge map. In actual applications, the above processing may be assigned to different program modules as needed, that is, the internal structure of the apparatus may be divided into different program modules to complete all or part of the method steps described above. In addition, the construction apparatus provided in the above embodiments and the construction method embodiments belong to the same idea. The individual components of the construction apparatus for the auxiliary diagnosis knowledge map should be understood as functional modules established to correspond to each step of the computer program for implementing the construction method. The specific implementation process is described in detail in the method embodiments and will not be repeated here.

It should be noted that in the auxiliary diagnosis method described in the foregoing embodiments, the method of obtaining the identification result of the target feature in the S04, that is, the method of analyzing the biological sample to obtain the identification result of the target feature, may not be limited to the method of the foregoing embodiments. The following is an explanation of another embodiment of the method of analyzing the biological sample to obtain the identification result of the target feature in the auxiliary diagnosis method.

The biological sample analysis apparatus that implements the biological sample analysis method may refer to a computer program product, such as various application programs, that is based on a computer program process to achieve the biological sample analysis function (i.e., to achieve the biological sample analysis method described in any of the following embodiments of the present disclosure); it may further refer to an auxiliary diagnosis apparatus loaded with a corresponding computer program product to achieve the biological sample analysis function (i.e., to achieve the biological sample analysis method described in any of the following embodiments of the present disclosure), such as various intelligent devices with storage and computing capabilities.

Referring to FIG. 19, FIG. 19 is a flowchart of a method for analyzing biological samples according to some embodiments of the present disclosure. The method includes, but is not limited to operations at blocks S602, S604, S606 and S608, as described in detail below.

At block S602: obtaining pulse data collected in each of different testing channels for a biological sample of a to-be-tested object.

The pulse data is an electrical pulse signal converted from the biological features of the biological sample tested by each testing channel. Taking the biological sample as a blood sample as an example, the biological features of the blood include the sizes of blood cells, the complexity of the cell contents, the nucleic acid content, etc.

Specifically, in some embodiments, taking a biological sample as a blood sample as an example, each testing channel may include, but is not limited to, an SFL (side florescence) channel for determining DNA/RNA content, an FSC (forward scatter) channel for determining cell volumes, an SSC (side scatter) channel for determining the complexity of the cells, a WBC channel for performing a white blood cell count, an RBC channel for performing a red blood cell count, a PLT channel for performing a platelet count, etc. In addition, in some embodiments, each testing channel may be arranged inside the biological sample analyzer, i.e., each pulse data is the raw data collected by the biological sample analyzer. In other embodiments, each testing channel may be arranged inside the biological sample analyzer that communicates with an auxiliary diagnosis apparatus, and the auxiliary diagnosis apparatus performs corresponding analysis based on each pulse data collected by the biological sample analyzer. It should be noted that although the description of the embodiments of the present disclosure takes a biological sample as a blood example as an example, it shall not constitute a limitation of the scope of protection of the present disclosure.

At block S604: for each of the pulse data of the different testing channels, quantifying pulse features of each of preset types, to obtain a first array corresponding to each pulse feature.

The pulse feature of the preset type refers to the pulse feature relevant to the corresponding analysis task of the biological sample, that is, the type of the pulse feature required for obtaining of the first array by quantization based on the corresponding analysis task. The pulse features of multiple preset types include but are not limited to pulse height, full-peak pulse width, first-half-peak pulse width, second-half-peak pulse width, pulse area, etc. Quantizing the pulse features of multiple preset types respectively refers to processes of expressing a value of the pulse feature of each corresponding type or the change of a value obtained by processing the value of the pulse feature of each corresponding type of pulse features in the form of a digital value. Each first array corresponding to the pulse feature of each preset type contains information of the pulse features of the preset type corresponding to the first array in the pulse data of each testing channel.

At block S606: fusing the first arrays corresponding to the pulse features of the multiple preset types to obtain a second array.

The fusion of the first arrays corresponding to the pulse features of the preset types means that the first arrays corresponding to the pulse features of the multiple preset types is superimposed in a specified dimensional direction to obtain a second array containing pulse feature information of multiple different preset types.

At block S608: extracting features from the second array and outputting an identification result of a target object (target feature) in the biological sample carried by the second array.

The target object refers to the object in the biological sample that is relevant to the current analysis task. It may be understood that the analysis task of the method for analyzing biological sample is to identify biological information features configured to determine types of diagnosis results in different biological samples. It can be seen that the original technical term "identification result of the target object" at block S608 refers to the biological information features determined according to the testing requirements of different biological samples, which has the same meaning as the identification result of the target feature at block S04. In the present disclosure, the original term "target object" may be uniformly replaced with "target feature". Taking a biological sample as a blood sample as an example, if it is currently necessary to analyze whether the Ig (immature granulocyte) in the to-be-tested blood is abnormal, then the target features include the content, volume, and/or distribution of the immature granulocytes. In some embodiments, depending on the current analysis task, the target features may include, but are not limited to, the quantities of different types of cells such as white blood cells, red blood cells, platelets, etc., or the contents, distributions, etc., of components of the biological sample such as hemoglobin, albumin, globulin, and DNA/RNA.

In some embodiments, features may be extracted from the second array by using an AI identification model to output the identification results of the target features in the biological sample carried by the second array. The AI identification model is an artificial intelligence (AI) model that uses machine learning to analyze the second array to extract features in the second array that are relevant to the target features, thereby outputting the identification results of the target features.

In the above embodiments, in the method for analyzing the biological sample, the pulse data of the biological sample of the to-be-tested object, that is collected by each testing channel is obtained, a pulse feature of each of multiple preset types carried by the pulse data of each testing channel is quantified respectively to obtain a first array for each pulse feature, the first arrays corresponding to the pulse features of the multiple preset types are fused to obtain a second array, features are extracted from the second array and the identification result of the target feature in the biological sample carried by the second array is outputted. The second array contains pulse features of multiple preset types carried by the pulse data of each different testing channel; in this way, the accuracy of obtaining the identification result based on the second array as the input data of the identification model is high.

In some other embodiments, features may be extracted from the second array by a conventional algorithm model to output the identification result of the target feature in the biological sample carried by the second array. The conventional algorithm may include image morphology algorithms, image classification algorithms, clustering algorithms, threshold segmentation algorithms, etc.

In some embodiments, the block S604 may further include operations: quantifying the pulse features of multiple the preset types carried by the pulse data of the different testing channels to obtain a two-dimensional first array corresponding to each testing channel. In some embodiments, a first dimension value of each two-dimensional first array in a first dimensional direction is determined based on the range of value of the pulse features of the multiple preset types, and a second dimension value of each two-dimensional first array in a second dimensional direction is determined based on the number of the testing channels.

In some embodiments, the S606 may further include: superimposing and fusing the first array corresponding to first pulse features and the two-dimensional first array corresponding to second pulse features in a third dimensional direction to obtain a three-dimensional second array of multi-features fusion, and a third-dimensional value of the three-dimensional second array in the third dimensional direction is determined based on the number of the pulse features of the multiple preset types.

In some embodiments, the S604 may further include: counting a frequency of the first pulse features of the preset type carried by the pulse data of each testing channel to obtain a first array of preset length corresponding to the first pulse features; and calculating the mean value at each frequency position of the second pulse features and the first pulse features to determine the first array corresponding to the second pulse features, based on the first array corresponding to the first pulse features and the second pulse features of the preset type.

In some embodiments, the operation of counting a frequency of the first pulse features of the preset type carried by the pulse data of each testing channel to obtain a first array of preset length corresponding to the first pulse features includes an operation of counting a frequency of the pulse height carried by the pulse data of each testing channel to obtain the first array corresponding to the pulse height.

In some embodiments, the operation of calculating the mean value at each frequency position of the second pulse features and the first pulse features to determine the first array corresponding to the second pulse features based on the first array corresponding to the first pulse features and the second pulse features of the preset type includes an operation of calculating, based on the first array corresponding to the pulse height of the pulse data and the full-peak width of the pulse data, the mean value at each frequency position of the full-peak width and the pulse height, and determining the first array corresponding to the full-peak width.

The second array obtained by fusing the first array corresponding to the pulse height and the first array corresponding to the full-peak width contains pulse height information and corresponding pulse width information for multiple different testing channels. Therefore, inputting the second array into the AI identification model may extract more features that are conducive to analysis tasks, and the accuracy of the analysis may be improved.

In some embodiments, the full-peak width may be divided into a first-half-peak width and a second-half-peak width i.e., the second pulse features include the first-half-peak width and the second-half-peak width. Then, the operation of calculating the mean value at each frequency position of the second pulse features and the first pulse features to determine the first array corresponding to the second pulse features based on the first array corresponding to the first pulse features and the second pulse features of the preset type includes operations: calculating, based on the first array corresponding to the pulse height of the pulse data and the first-half-peak width of the pulse data, the mean value at each frequency position of the first-half-peak width and the pulse height, and determining the first array corresponding to the first-half-peak width; and calculating, based on the first array corresponding to the pulse height of the pulse data and the second-half-peak width of the pulse data, the mean value at each frequency position of the second-half-peak width and the pulse height and determining the first array corresponding to the second-half-peak width.

In addition, in other embodiments of the present disclosure, the operation of calculating the mean value at each frequency position of the second pulse features and the first pulse features to determine the first array corresponding to the second pulse features based on the first array corresponding to the first pulse features and the second pulse features of the preset type may further include an operation of calculating, based on the first array corresponding to the pulse height of the pulse data and the pulse area of the pulse data, the mean value at each frequency position of the pulse area and the pulse height, and determining the first array corresponding to the pulse area.

In the S606, the obtained second array not only includes the pulse height information that contributes significantly to the analysis and identification of the biological samples, but also includes information such as full-peak width, first-half-peak width, second-half-peak width, pulse area, etc., which also contributes to the analysis and identification of the biological samples, thereby improving the accuracy of the identification results in the S608.

In some embodiments, before the operation of counting a frequency of the first pulse features of the preset type carried by the pulse data of each testing channel to obtain a first array of preset length corresponding to the first pulse features, the method further includes operations: setting length values based on the height range of the pulse height.

In some embodiments, taking the biological sample as a blood sample as an example, the S602 may further include operations: obtaining cell pulse signals of the biological sample cells of the to-be-tested object that are collected by multiple testing channels. Each cell pulse signal is an electrical pulse signal converted from the biological feature of a corresponding cell.

In some embodiments, the S602 may further include: acquiring apparatus-information-file data of the biological sample analyzer; determining, based on the apparatus-information-file data, multiple target testing channels of the biological sample cells of the to-be-tested object; and obtaining the cell pulse signals collected by the multiple target testing channels.

In some embodiments, the S602 may further include: obtaining sample detection data output by the biological sample analyzer; and obtaining, based on the sample detection data, the pulse data of the biological sample of the to-be-tested object that is collected in the SFL channel, the FSC channel, the SSC channel, the WBC channel, the PLT channel, and the RBC channel. Herein, biological sample analyzer is configured with a combination of at least two testing channels selected from the group consisting of: the SFL channel, FSC channel, the SSC channel, the WBC channel, the PLT channel, and the RBC channel.

In some embodiments, the S602 may further include: obtaining sample detection data output by the biological sample analyzer; and obtaining, based on the sample detection data, the pulse data of the biological sample of the to-be-tested object that is collected in the SFL channel, the FSC channel, the SSC channel, the WBC channel, the PLT channel, and the RBC channel. Herein, biological sample analyzer is configured with a combination of at least two testing channels selected from the group consisting of: the SFL channel, FSC channel, the SSC channel, the WBC channel, the PLT channel, and the RBC channel.

The S604 may further include: counting the frequency of the first pulse features of the preset types carried by the pulse data of each testing channel, and obtaining the first array of preset length value corresponding to the first pulse features; calculating, based on the first array corresponding to the pulse height of the pulse data and the first-half-peak width of the pulse data, the mean value at each frequency position of the first-half-peak width and the pulse height, and determining the first array corresponding to the first-half-peak width; and calculating, based on the first array corresponding to the pulse height of the pulse data and the second-half-peak width of the pulse data, the mean value at each frequency position of the second-half-peak width and the pulse height and determining the first array corresponding to the second-half-peak width.

An alarm for the abnormal target feature is formed based on the identification result of the abnormal target feature. In some embodiments, the AI identification model is a convolutional neural network model such as ResNet, VGG, etc. In other embodiments, the AI identification model may also be other models constructed by using one-dimensional convolution.

Referring to FIG. 20, in some embodiments, the present disclosure further provides a biological sample analysis apparatus, including: an obtaining module 601, a quantification module 602, a fusion module 603, and an identification module 604. The obtaining module 601 is configured to obtain pulse data collected in each of different testing channels for a biological sample of a to-be-tested object. The quantification module 602 is configured to, for each of the pulse data of the different testing channels, quantify pulse features of each of preset types, to obtain a first array corresponding to each pulse feature. The fusion module 603 is configured to fuse the first arrays corresponding to the pulse features of the preset types to obtain a second array. The identification module 604 is configured to extract features from the second array through an AI identification model and output an identification result of a target feature in the biological sample carried by the second array.

It should be noted that the biological sample analysis apparatus provided in the above embodiments is only exemplified by the above division of program modules in the process of implementing the biological sample analysis method. In actual applications, the above processing may be assigned to different program modules as needed, that is, the internal structure of the apparatus may be divided into different program modules to complete all or part of the method steps described above. In addition, the biological sample analysis apparatus provided in the above embodiments and the embodiments of the biological sample analysis method belong to the same idea. Each component of the biological sample analysis apparatus should be understood as a functional module established corresponding to each step of the computer program for implementing the construction method. The specific implementation process is described in detail in the method embodiments and will not be repeated here.

The above is only some specific embodiments of the present disclosure, but the scope of the present disclosure is not limited thereto. Any changes or substitutions that may be readily conceived within the technical scope disclosed in the present disclosure by those skilled in the art shall be covered by the scope of the present disclosure. The scope of the present disclosure shall be object to the protection scope of the claims.

**Claims**

1. An auxiliary diagnosis apparatus, **characterized by** comprising:

   an obtaining module, configured to obtain detection parameters corresponding to a biological sample of a to-be-tested object and an identification result of a target feature of the biological sample of the to-be-tested object;
   a similar feature determination module, configured to search from a sample feature library containing sample features corresponding to historical diagnosis cases according to the identification result of the target feature, for determining similar sample feature information corresponding to the identification result of the target feature;
   an auxiliary decision-making module, configured to determine an auxiliary diagnosis result of the to-be-tested object based on the detection parameters, the identification result of the target feature, and the similar sample feature information; and
   an output module, configured to output an auxiliary diagnosis report containing the auxiliary diagnosis result and a diagnosis basis characterizing the auxiliary diagnosis result; wherein the diagnosis basis comprises a sample analysis image characterizing an abnormality of the target feature in the identification result of the target feature and a similar sample image characterizing an abnormality of the target feature in the similar sample feature information.

2. The auxiliary diagnosis apparatus as claimed in claim 1, wherein the obtaining module is further configured to obtain clinical information data of the to-be-tested object;

   the auxiliary decision-making module is specifically configured to determine the auxiliary diagnosis result of the to-be-tested object based on the clinical information data, the detection parameters, the identification result of the target feature, and the similar sample feature information of the to-be-tested object;
   wherein the detection parameters comprise at least one of the following:

   test report parameters or abnormal sample parameters output by a sample analyzer that performs a test on the biological sample of the to-be-tested object;
   research parameters of the biological sample of the to-be-tested object;
   the status parameters of a testing device that performs the test on the biological sample of the to-be-tested object.

3. The auxiliary diagnosis apparatus as claimed in claim 1, wherein the obtaining module is specifically configured to:

obtain abnormality warning information characterizing an abnormality of the target feature, and a sample analysis image carrying an abnormality marker of the target feature;
the auxiliary decision-making module is specifically configured to:

according to the sample analysis image carrying the abnormality marker of the target feature, search and determine a similar sample image from a sample image library containing sample analysis images corresponding to the historical diagnosis cases; wherein the sample analysis images corresponding to the historical diagnosis cases in the sample image library have been confirmed by microscopy; and according to the abnormality warning information, search and determine a similar microscopic image from a microscopic image library containing microscopic images corresponding to the historical diagnosis cases.

4. The auxiliary diagnosis apparatus as claimed in claim 3, wherein the auxiliary diagnosis report further comprises an activation map that is determined based on the abnormality warning information and that prominently displays an abnormal feature region;

the activation map is obtained by superimposing a heat map obtained based on a feature map of the sample analysis image on the sample analysis image; wherein the heat map characterizes a decision-making influence of each cell region of the sample analysis image on determining first abnormal feature information;
the sample analysis image carries a positioning marker; wherein the positioning marker is formed by a highlighted region based on the activation map and is configured to indicate a location of the identification result of the target feature in the sample analysis image.

5. The auxiliary diagnosis apparatus as claimed in claim 2, wherein the similar sample image comprises:
according to clinical information data of the to-be-tested object, determine a target object type of the to-be-tested object; and search and determine a similar abnormal sample image with a highest similarity and a similar normal sample image with a highest similarity from the sample image library corresponding to the target object type.

6. The auxiliary diagnosis apparatus as claimed in claim 3, wherein the sample feature library further comprises at least one of the following sample feature information:

a device status reference feature that characterizes whether the status of a testing device is abnormal, an abnormal sample reference feature that characterizes whether the quality of the biological sample is abnormal, and an identification result reference feature that characterizes whether the identification result of the target feature is abnormal;
the similar feature determination module is specifically configured to:
according to the identification result of the target feature, search from the sample feature library and determine the device status reference feature, the abnormal sample reference feature, and/or the identification result reference feature corresponding to the identification result of the target feature.

7. The auxiliary diagnosis apparatus as claimed in claim 1, wherein the obtaining module is specifically configured to:

extract a sample analysis image from the detection parameters corresponding to the biological sample of the to-be-tested object;
input the sample analysis image into an image analysis model, perform feature extraction and identification on the sample analysis image through the image analysis model, and obtain the identification result of the target feature of the biological sample;
wherein the identification result of the target feature comprises a sample analysis image carrying an abnormality marker and abnormality warning information; or, the identification result of the target feature comprises abnormality warning information, a sample analysis image carrying an abnormality marker, and an activation map that is determined based on the abnormality warning information and that prominently displays an abnormal feature region.

8. The auxiliary diagnosis apparatus as claimed in claim 1, wherein the auxiliary decision-making module is further configured to determine the auxiliary diagnosis result of the to-be-tested object based on a constructed knowledge map;
wherein a construction process of the knowledge map comprises:

classifying the historical diagnosis cases according to preset diagnosis result types;
according to clinical information data of tested objects in the historical diagnosis cases corresponding to each diagnosis result type, detection parameters corresponding to the tested objects, and identification results of target features of biological samples corresponding to the tested objects, determining a criterion rule corresponding to each diagnosis result type based on data mining and/or an expert consensus database; and
constructing an auxiliary diagnosis knowledge map according to the criterion rule.

9. The auxiliary diagnosis apparatus as claimed in claim 8, wherein the output module is further configured to combine the clinical information data, the detection parameters, and the identification result of the target feature of the to-be-tested object with corresponding criterion rules in different detection modes, and output the auxiliary diagnosis report containing the auxiliary diagnosis results of the to-be-tested object in different testing modes and the diagnosis basis that characterizes the auxiliary diagnosis results.

10. The auxiliary diagnosis apparatus as claimed in claim 8, wherein the auxiliary decision-making module is further configured to:

construct a first determination condition according to the clinical information data of the to-be-tested object;
construct a second determination condition according to the detection parameters of the to-be-tested object;
construct a third determination condition according to the identification result of the target feature of the biological sample of the to-be-tested object; and
input the first determination condition, the second determination condition, and the third determination condition into the knowledge map in parallel, and determine, through the knowledge map, the auxiliary diagnosis result of the to-be-tested object according to a compliance of the first determination condition, the second determination condition, and the third determination condition with the criterion rule of each diagnosis result type.

11. The auxiliary diagnosis apparatus as claimed in claim 8, wherein the determining a criterion rule corresponding to each diagnosis result type based on data mining and/or an expert consensus database comprises:

according to the clinical information data of the tested objects in the historical diagnosis cases corresponding to each diagnosis result type, the detection parameters corresponding to the tested objects, and the identification results of the target features of the biological samples corresponding to the tested objects, individually constructing a sample dataset with each diagnosis result type as a classification label;
performing feature selection on candidate features in each sample dataset according to a classification contribution, and output a target feature result corresponding to a corresponding classification label and taking the target feature result as the criterion rule corresponding to a corresponding diagnosis result type.

12. The auxiliary diagnosis apparatus as claimed in claim 11, wherein constructing an auxiliary diagnosis knowledge map according to the criterion rule comprises:

parsing the criterion rule to form a key-value pair for the criterion rule, where the key is a corresponding diagnosis result type and the value is a decision condition set corresponding to the corresponding diagnosis result type; and
forming a key-value pair database according to the key-value pair corresponding to the criterion rule for each diagnosis result type, and constructing the auxiliary diagnosis knowledge map according to the key-value pair database.

13. The auxiliary diagnosis apparatus as claimed in any of claims 1 to 12, wherein,

in response to the identification result of the target feature being of a first type, the auxiliary diagnosis report comprises at least two similar sample images whose similarity satisfies a preset condition, and abnormality levels of the at least the two similar sample images are the same;
in response to the identification result of the target feature being of a second type, the auxiliary diagnosis report comprises a similar sample image with a highest similarity and at least one reference sample image, and abnormality levels of the similar sample image and the at least one reference sample image are different.

14. The auxiliary diagnosis apparatus as claimed in claim 13, wherein the auxiliary diagnosis apparatus further comprises:
an image matching module, configured to determine a matched image set in the sample image library according to a category of the identification result of the target feature; determine the similar sample image whose similarity satisfies the preset condition based on a similarity between the sample analysis image and a reference image in the matched image set; and according to a mode category to which the similar sample image belongs, determine the reference sample image in the sample image library belonging to the mode category same as the similar sample image and whose abnormality level is different from the abnormality level of the similar sample image.

15. The auxiliary diagnosis apparatus as claimed in claim 1, wherein the auxiliary diagnosis report further comprises:

a reference sample image that matches the sample analysis image and has a different abnormality level from the similar sample image;
wherein the abnormality level of the similar sample image is one of severe, moderate, and mild, and the abnormality level of the reference sample image comprises the other two of severe, moderate, and mild.

16. The auxiliary diagnosis apparatus as claimed in claim 1, wherein a type of the auxiliary diagnosis result comprises a first result type with an explicit risk result and a second result type with an implicit risk result; the output module is further configured to:

in response to the auxiliary diagnosis result being of the first result type, display the explicit risk result through a result display region of the auxiliary diagnosis report, display a key evidence that led to the explicit risk result and a subsequent processing recommendation through an analysis display region of the auxiliary diagnosis report, and display the sample analysis image, the similar sample image, an activation map, and a similar microscopic image through an image display region of the auxiliary diagnosis report;
in response to the auxiliary diagnosis result being of the second result type, display abnormality warning information in the identification result of the target feature through the result display region of the auxiliary diagnosis report, display a maximum suspected risk corresponding to the abnormality warning information and a subsequent processing recommendation through the analysis display region of the auxiliary diagnosis report, and display the sample analysis image, the similar sample image, the activation map, and the similarity microscopic image through the image display region of the auxiliary diagnosis report.

17. The auxiliary diagnosis apparatus as claimed in claim 1, wherein the diagnosis basis further comprises at least one of the following: a device status reference feature that characterizes whether the status of a testing device corresponding to the auxiliary diagnosis result is abnormal, an abnormal sample reference feature that characterizes whether the quality of the biological sample is abnormal, and an identification result reference feature that characterizes whether the identification result of the target feature is abnormal;
the output module is further configured to display the device status reference feature, the abnormal sample reference feature, and/or the identification result reference feature corresponding to the auxiliary diagnosis result in the analysis display region of the auxiliary diagnosis report.

18. The auxiliary diagnosis apparatus as claimed in claim 1, wherein the auxiliary diagnosis report output by the output module further comprises a corresponding conventional test result of the to-be-tested object and a diagnosis basis of the conventional test result; wherein the conventional test result comprises abnormality warning information obtained according to a conventional test method, and the diagnosis basis of the conventional test result comprises at least a sample analysis image of the biological sample of the to-be-tested object.

19. The auxiliary diagnosis apparatus as claimed in claim 1, wherein the obtaining module is specifically configured to

obtain pulse data collected in each of different testing channels for the biological sample of the to-be-tested object;
for each of the pulse data of the different testing channels, quantify pulse features of each of preset types, to obtain a first array corresponding to each pulse feature;
fuse the first arrays corresponding to the pulse features of the preset types to obtain a second array; and
extract features from the second array, and output the identification result of the target feature in the biological sample carried by the second array.

20. An auxiliary diagnosis apparatus, **characterized by** comprising:

an obtaining module, configured to obtain detection parameters corresponding to a biological sample of a to-be-tested object and an identification result of a target feature of the biological sample of the to-be-tested object;
a similar feature determination module, configured to search from a sample feature library containing sample features corresponding to historical diagnosis cases according to the identification result of the target feature, for determining similar sample feature information corresponding to the identification result of the target feature;

an auxiliary decision-making module, configured to determine an auxiliary diagnosis result of the to-be-tested object based on the detection parameters, the identification result of the target feature, and the similar sample feature information, and

an output module, configured to output an auxiliary diagnosis report containing the auxiliary diagnosis result and a diagnosis basis characterizing the auxiliary diagnosis result; wherein the diagnosis basis comprises a sample analysis image characterizing an abnormality of the target feature in the identification result of the target feature and a device status reference feature characterizing whether the status of a testing device is abnormal in the similar sample feature information.

21. An apparatus for constructing an auxiliary diagnosis knowledge map, **characterized by** comprising:

a dataset construction module, configured to construct sample datasets with target identification categories as classification labels according to object attribute data in historical diagnosis samples and sample analysis data of a sample analyzer;

a feature selection module, configured to perform feature selection on candidate features in each sample dataset according to a classification contribution through a feature selection model, and output a target feature result corresponding to each classification label;

a map construction module, configured to form a category identification rule according to the target feature result corresponding to each classification label, and construct the auxiliary diagnosis knowledge map according to the category identification rule.

22. The apparatus as claimed in claim 21, wherein the dataset construction module is specifically configured to:

obtain the historical diagnosis samples corresponding to different target identification categories; and

cluster the historical diagnosis samples of a same target identification category to form a plurality of sample datasets each corresponding to a corresponding target identification category, wherein each sample dataset is classified with a corresponding target identification category as a corresponding classification label.

23. The apparatus as claimed in claim 22, wherein the obtaining the historical diagnosis samples corresponding to different target identification categories comprises:

obtaining the object attribute data of a historical test object from a laboratory information system according to a historical diagnosis record;

obtaining the sample analysis data for a test sample from the sample analyzer for the historical test object;

obtaining the target identification category corresponding to the historical diagnosis record; and

forming a historical diagnosis sample corresponding to the target identification category according to the object attribute data and the sample analysis data of the historical test object.

24. The apparatus as claimed in claim 21, wherein the dataset construction module is specifically configured to:

perform a testing analysis on image data in the sample analysis data of the sample analyzer through an image identification model, for obtaining a target feature identification result corresponding to the image data; and

construct a sample dataset for each historical diagnosis sample, with the object attribute data contained in the historical diagnosis sample, the sample analysis data of the sample analyzer for the test sample, and the target feature identification result as candidate features, and the target identification category corresponding to the historical diagnosis sample as the classification label.

25. The apparatus as claimed in in claim 24, wherein the performing a testing analysis on image data in the sample analysis data of the sample analyzer through an image identification model, for obtaining a target feature identification result corresponding to the image data comprises:

inputting the image data into the image identification model, performing feature extraction and identification on the image data through the image identification model, and obtaining the target feature identification result corresponding to the image data; wherein the target feature identification result comprises abnormal warning data and a sample analysis image carrying an abnormality marker.

26. The apparatus as claimed in in claim 21, wherein the feature selection module is specifically configured to:

sort the candidate features in the sample datasets according to a classification contribution through a decision tree model, and output the target feature results corresponding to the classification labels corresponding to the sample datasets according to a sorting result; or,
sort the candidate features in the sample datasets according to a classification contribution through a random forest model, and output the target feature results corresponding to the classification labels corresponding to the sample datasets according to a sorting result.

27. The apparatus as claimed in in claim 26, wherein the map construction module is specifically configured to:

according to the target feature result corresponding to each classification label, form the category identification rule for the classification label based on a correspondence between a target feature, that meets a preset requirement for the classification contribution in a classification contribution ranking or meets a preset condition for a ranking position in the classification contribution ranking, and the classification label; and
construct the auxiliary diagnosis knowledge map according to the category identification rule for the classification label.

28. A biological sample analysis apparatus, **characterized by** comprising:

an obtaining module, configured to obtain pulse data collected in each of different testing channels for a biological sample of a to-be-tested object;
a quantification module, configured to, for each of the pulse data of the different testing channels, quantify pulse features of each of preset types, to obtain a first array corresponding to each pulse feature;
a fusion module, configured to fuse the first arrays corresponding to the pulse features of the preset types to obtain a second array; and
an identification module, configured to extract features from the second array and output an identification result of a target feature in the biological sample carried by the second array.

29. An auxiliary diagnosis method, **characterized by** comprising:

obtaining detection parameters corresponding to a biological sample of a to-be-tested object and an identification result of a target feature of the biological sample of the to-be-tested object;
searching from a sample feature library containing sample features corresponding to historical diagnosis cases according to the identification result of the target feature, for determining similar sample feature information corresponding to the identification result of the target feature;
determining an auxiliary diagnosis result of the to-be-tested object based on the detection parameters, the identification result of the target feature, and the similar sample feature information; and
outputting an auxiliary diagnosis report containing the auxiliary diagnosis result and a diagnosis basis characterizing the auxiliary diagnosis result; wherein the diagnosis basis comprises a sample analysis image characterizing an abnormality of the target feature in the identification result of the target feature and a similar sample image characterizing an abnormality of the target feature in the similar sample feature information.

30. A sample analyzer, **characterized by** comprising:

a sampling assembly, configured to collect and dispense a biological sample of a to-be-tested object; wherein the biological sample is a blood sample;
a reaction assembly, configured to process the biological sample to form a to-be-tested solution;
a drive assembly, configured to drive a liquid path between the sampling assembly and the reaction assembly;
a detection assembly, configured to classify and count blood cells contained in the to-be-tested solution to obtain sample detection data comprising sample detection parameters and a sample analysis image; and
the auxiliary diagnosis apparatus as claimed in any of claims 1 to 20, configured to output the auxiliary diagnosis report based on the sample detection data.

31. A sample analysis system, comprising the auxiliary diagnosis apparatus as claimed in any of claims 1 to 20 and a stand-alone sample analyzer or a cascade sample analyzer connected to the auxiliary diagnosis apparatus.

11

21

Sample analyzer → Auxiliary diagnosis apparatus

FIG.1

21

Sample analyzer

11

Auxiliary diagnosis apparatus

FIG.2

obtaining detection parameters corresponding to a biological sample of a to-be-tested object — S02

obtaining an identification result of a target feature of the biological sample of the to-be-tested object — S04

based on the identification result of the target feature, performing a search in a sample feature library containing sample features corresponding to historical diagnosis cases to determine similar sample feature information corresponding to the identification result of the target feature — S06

determining an auxiliary diagnosis result for the to-be-tested object based on the detection parameters, the identification result of the target feature, and the similar sample feature information, and outputting an auxiliary diagnosis report containing the auxiliary diagnosis result and a diagnosis basis characterizing the auxiliary diagnosis result; where the diagnosis basis includes: first feature information corresponding to the identification result of the target feature indicating an abnormality of the target feature, and second feature information corresponding to the similar sample feature information indicating an abnormality of the target feature — S08

FIG.3

AI Analysis

1、XXX（Auxiliary diagnosis category information）

AI analysis details

1、XXX（Abnormal condition 1）
2、XXX（Abnormal condition 2）
3、XXX（Suggestions）

Blast activation map    Patient scatter plot    No-Blast microscopic reference image

Best-fit image (reference image for heavy Blast microscopy)    Reference image for light Blast microscopy    Reference image for medium Blast microscopy

Other notes

Patient age: XX; gender: male;
sample type: venous whole blood

1. White blood cells significantly elevated, platelets reduced, moderate levels of blast cells with a large number of immature granulocytes. High possibility of disease A
2. Recommended to perform tests such as XXX to confirm the diagnosis.
3. Please consult a doctor.

Diagnostic basis:
WBC: XX (reference range: YY)
PLT: XX (reference range: YY)
IG: XX (reference range: YY)
Blast cells: XX (reference range: YY)

Patient scatter plot

Similar scatter plot and corresponding similar microscopic image

Patient scatter plot confirmed by microscopic examination

Reference image of immature granulocytes (IG) seen under the microscope

FIG.4

obtaining detection parameters corresponding to a biological sample of a to-be-tested object ⟩S02

obtaining an identification result of a target feature of the biological sample of the to-be-tested object ⟩S04

according to the sample analysis image carrying the abnormality marker of the target feature, searching and determining a similar sample image from a sample image library containing sample analysis images corresponding to the historical diagnosis cases ⟩S061

according to the abnormality warning information, searching and determining a similar microscopic image from a microscopic image library containing microscopic images corresponding to the historical diagnosis cases ⟩S062

determining an auxiliary diagnosis result for the to-be-tested object based on the detection parameters, the identification result of the target feature, and the similar sample feature information, and outputting an auxiliary diagnosis report containing the auxiliary diagnosis result and a diagnosis basis characterizing the auxiliary diagnosis result; where the diagnosis basis includes: first feature information corresponding to the identification result of the target feature indicating an abnormality of the target feature, and second feature information corresponding to the similar sample feature information indicating an abnormality of the target feature ⟩S08

FIG.5

obtaining detection parameters corresponding to a biological sample of a to-be-tested object ⟩S02

obtaining an identification result of a target feature of the biological sample of the to-be-tested object ⟩S04

classifying each test object according to clinical information data of the test object, and establishing the sample feature library corresponding to each object type according to the object type and the historical diagnosis cases ⟩S05

according to the sample analysis image carrying the abnormality marker of the target feature, searching and determining a similar sample image from a sample image library containing sample analysis images corresponding to the historical diagnosis cases ⟩S061

according to the abnormality warning information, searching and determining a similar microscopic image from a microscopic image library containing microscopic images corresponding to the historical diagnosis cases ⟩S062

determining an auxiliary diagnosis result for the to-be-tested object based on the detection parameters, the identification result of the target feature, and the similar sample feature information, and outputting an auxiliary diagnosis report containing the auxiliary diagnosis result and a diagnosis basis characterizing the auxiliary diagnosis result; where the diagnosis basis includes: first feature information corresponding to the identification result of the target feature indicating an abnormality of the target feature, and second feature information corresponding to the similar sample feature information indicating an abnormality of the target feature ⟩S08

FIG.6

obtaining detection parameters corresponding to a biological sample of a to-be-tested object — S02

↓

obtaining an identification result of a target feature of the biological sample of the to-be-tested object — S04

↓

according to the sample analysis image carrying the abnormality marker of the target feature, searching and determining a similar sample image from a sample image library containing sample analysis images corresponding to the historical diagnosis cases — S061

↓

according to the abnormality warning information, searching and determining a similar microscopic image from a microscopic image library containing microscopic images corresponding to the historical diagnosis cases — S062

↓

According to the target feature identification result, determining a similar sample image that matches the target feature identification result from the sample image library, and determine a similar mirror image associated with the similar sample image from the microscopic image library — S063

↓

determining an auxiliary diagnosis result for the to-be-tested object based on the detection parameters, the identification result of the target feature, and the similar sample feature information, and outputting an auxiliary diagnosis report containing the auxiliary diagnosis result and a diagnosis basis characterizing the auxiliary diagnosis result; where the diagnosis basis includes: first feature information corresponding to the identification result of the target feature indicating an abnormality of the target feature, and second feature information corresponding to the similar sample feature information indicating an abnormality of the target feature — S08

FIG.7

according to the clinical information data of the to-be-tested object, determining from the historical diagnosis cases target historical diagnosis cases of test objects with the same object type as the to-be-tested object — S0601

↓

according to sample images of the target historical diagnosis cases, forming a sample image library; and according to microscopic images of the target historical diagnosis cases, forming a microscopic image library — S0602

↓

according to the sample analysis image carrying the abnormality marker of the target feature, searching and determining a similar sample image from the sample image library containing sample analysis images corresponding to the historical diagnosis cases — S061

↓

according to the abnormality warning information, searching and determining a similar microscopic image from the microscopic image library containing microscopic images corresponding to the historical diagnosis cases — S062

FIG.8

obtaining detection parameters corresponding to a biological sample of a to-be-tested object ⟋S02

extracting a biological sample analysis image from the detection parameters corresponding to the biological sample of the to-be-tested object ⟋S041

inputting the biological sample analysis image to an image analysis model, performing feature extraction and identification on the biological sample analysis image through the image analysis model, and obtaining the identification result of the target feature of the biological sample; where the identification result of the target feature includes the sample analysis image carrying an abnormality marker and the abnormality warning information; or, the identification result of the target feature includes the abnormality warning information, the sample analysis image carrying the abnormality marker, and an activation map that is determined based on the abnormality warning information and that prominently displays an abnormal feature region ⟋S042

according to the sample analysis image carrying the abnormality marker of the target feature, searching and determining a similar sample image from a sample image library containing sample analysis images corresponding to the historical diagnosis cases ⟋S061

according to the abnormality warning information, searching and determining a similar microscopic image from a microscopic image library containing microscopic images corresponding to the historical diagnosis cases ⟋S062

determining an auxiliary diagnosis result for the to-be-tested object based on the test report parameters, the target feature identification result, the similar sample image and/or the similar microscopic image, and outputting an auxiliary diagnosis report containing the auxiliary diagnosis result and a diagnosis basis characterizing the auxiliary diagnosis result; where the diagnosis basis includes: first feature information corresponding to the identification result of the target feature indicating an abnormality of the target feature, second feature information corresponding to the similar sample feature information indicating an abnormality of the target feature, and/or third feature information corresponding to the similar mirror image indicating the abnormality degree ⟋S08

FIG.9

obtaining detection parameters corresponding to a biological sample of a to-be-tested object $\quad$ S02

obtaining an identification result of a target feature of the biological sample of the to-be-tested object $\quad$ S04

based on the identification result of the target feature, performing a search in a sample feature library containing sample features corresponding to historical diagnosis cases to determine similar sample feature information corresponding to the identification result of the target feature $\quad$ S06

classifying the historical diagnosis cases according to preset diagnosis result types $\quad$ S071

according to clinical information data of the tested objects in the historical diagnosis cases corresponding to each diagnosis result type, detection parameters corresponding to the tested objects, and the identification results of the target feature of the biological samples corresponding to the tested objects, determining a criterion rule corresponding to each diagnosis result type based on data mining and/or an expert consensus database $\quad$ S072

constructing an auxiliary diagnosis knowledge map according to the criterion rule $\quad$ S073

determining an auxiliary diagnosis result for the to-be-tested object based on the test report parameters, the target feature identification result, the similar sample image and/or the similar microscopic image, and outputting an auxiliary diagnosis report containing the auxiliary diagnosis result and a diagnosis basis characterizing the auxiliary diagnosis result; where the diagnosis basis includes: first feature information corresponding to the identification result of the target feature indicating an abnormality of the target feature, second feature information corresponding to the similar sample feature information indicating an abnormality of the target feature, and/or third feature information corresponding to the similar mirror image indicating the abnormality degree $\quad$ S08

FIG.10

| obtaining detection parameters corresponding to a biological sample of a to-be-tested object | S02 |

↓

| extracting a biological sample analysis image from the detection parameters corresponding to the biological sample of the to-be-tested object | S041 |

↓

| inputting the biological sample analysis image to an image analysis model, performing feature extraction and identification on the biological sample analysis image through the image analysis model, and obtaining the identification result of the target feature of the biological sample; where the identification result of the target feature includes the sample analysis image carrying an abnormality marker and the abnormality warning information; or, the identification result of the target feature includes the abnormality warning information, the sample analysis image carrying the abnormality marker, and an activation map that is determined based on the abnormality warning information and that prominently displays an abnormal feature region | S042 |

↓

| according to the sample analysis image carrying the abnormality marker of the target feature, searching and determining a similar sample image from a sample image library containing sample analysis images corresponding to the historical diagnosis cases | S061 |

↓

| according to the abnormality warning information, searching and determining a similar microscopic image from a microscopic image library containing microscopic images corresponding to the historical diagnosis cases | S062 |

↓

| determining the auxiliary diagnosis result of the to-be-tested object according to the clinical information data, the detection parameters, the identification result of the target feature, and the similar sample feature information of the to-be-tested object | S081 |

↓

| according to a result type of the auxiliary diagnosis result, outputting the auxiliary diagnosis report matching the result type | S082 |

FIG.11

Result display region

Analysis display region

Conventional test result display region

Image display region

FIG.12

Auxiliary diagnosis apparatus

First obtaining module ⌇ 101

Second obtaining module ⌇ 102

⌇ 100

Similar feature determination module ⌇ 103

Auxiliary decision module ⌇ 104

Output module ⌇ 105

FIG.13

constructing sample datasets with target identification categories as classification labels according to object attribute data in historical diagnosis samples and sample analysis data of a sample analyzer  S502

performing feature selection on candidate features in each sample dataset according to a classification contribution through a feature selection model, and outputting a target feature result corresponding to each classification label  S504

forming a category identification rule according to the target feature result corresponding to each classification label, and constructing the auxiliary diagnosis knowledge map according to the category identification rule  S506

FIG.14

| obtaining the historical diagnosis samples corresponding to different target identification categories | S5021 |

↓

| clustering the historical diagnosis samples of a same target identification category to form a plurality of sample datasets each corresponding to a corresponding target identification category, where each sample dataset is classified with a corresponding target identification category as a corresponding classification label | S5022 |

↓

| performing feature selection on candidate features in each sample dataset according to a classification contribution through a feature selection model, and outputting a target feature result corresponding to each classification label | S504 |

↓

| forming a category identification rule according to the target feature result corresponding to each classification label, and constructing the auxiliary diagnosis knowledge map according to the category identification rule | S506 |

FIG.15

| obtaining the object attribute data of a historical test object from a laboratory information system according to a historical diagnosis record | S50211 |

↓

| obtaining the sample analysis data from the sample analyzer for the historical test object | S50212 |

↓

| obtaining the target identification category of the historical test object, and forming a historical diagnosis sample corresponding to the target identification category according to the object attribute data and the sample analysis data of the historical test object | S50213 |

FIG.16

| performing a testing analysis on image data in the sample analysis data of the sample analyzer through an image identification model, for obtaining a target feature identification result corresponding to the image data | S5021a |

↓

| constructing a sample dataset for each historical diagnosis sample, with the object attribute data contained in the historical diagnosis sample, the sample analysis data of the sample analyzer for the test sample, and the target feature identification result as candidate features, and the target identification category corresponding to the historical diagnosis sample as the classification label | S5022a |

↓

| performing feature selection on candidate features in each sample dataset according to a classification contribution through a feature selection model, and outputting a target feature result corresponding to each classification label | S504 |

↓

| forming a category identification rule according to the target feature result corresponding to each classification label, and constructing the auxiliary diagnosis knowledge map according to the category identification rule | S506 |

FIG.17

Apparatus for constructing auxiliary
diagnosis knowledge map

Dataset construction module — 501

Feature selection module — 502

Map construction module — 503

FIG. 18

obtaining pulse data collected in each of different testing channels for a biological sample of a to-
be-tested object — S602

quantifying, for each of the pulse data of the different testing channels, pulse features of each of
preset types, to obtain a first array corresponding to each pulse feature — S604

fusing the first arrays corresponding to the pulse features of the multiple preset types to obtain a
second array — S606

extracting features from the second array and outputting an identification result of a target feature
in the biological sample carried by the second array — S608

FIG. 19

Biological sample analysis apparatus

Obtaining module — 101

Quantification module — 102

Fusion module — 103

Identification module — 104

FIG. 20

| | | |
|---|---|---|
| <div align="center">**INTERNATIONAL SEARCH REPORT**</div> | International application No. | |
| | **PCT/CN2023/085278** | |

**A. CLASSIFICATION OF SUBJECT MATTER**

G16H 50/70(2018.01)i

According to International Patent Classification (IPC) or to both national classification and IPC

**B. FIELDS SEARCHED**

Minimum documentation searched (classification system followed by classification symbols)

IPC: G16H; G01N

Documentation searched other than minimum documentation to the extent that such documents are included in the fields searched

Electronic data base consulted during the international search (name of data base and, where practicable, search terms used)

CNABS; CNTXT; VEN; USTXT; EPTXT; WOTXT; CNKI; IEEE: 辅助诊断, 样本, 图像, 相似, 参数, 知识图谱, 特征, 贡献度, 脉冲, 融合, 分析, assisted diagnostic, sample, image, similar, parameter, knowledge graph, feature, contribution, pulse, fusion, analysis

**C. DOCUMENTS CONSIDERED TO BE RELEVANT**

| Category* | Citation of document, with indication, where appropriate, of the relevant passages | Relevant to claim No. |
|---|---|---|
| X | CN 111785363 A (CIXI INSTITUTE OF BIOMEDICAL ENGINEERING, NINGBO INSTITUTE OF INDUSTRIAL TECHNOLOGY, CHINESE ACADEMY OF SCIENCES et al.) 16 October 2020 (2020-10-16)<br>description, paragraphs 19-29 | 1-18, 20, 29-31 |
| X | CN 107194158 A (SHENZHEN MGENE TECHNOLOGY CO., LTD.) 22 September 2017 (2017-09-22)<br>description, paragraphs 50-139 | 1-18, 20, 29-31 |
| Y | CN 110911009 A (NANJING MEDICAL UNIVERSITY) 24 March 2020 (2020-03-24)<br>description, paragraphs 71-130 | 21-27 |
| Y | CN 109346169 A (HANCLOUDCLINIC LIMITED) 15 February 2019 (2019-02-15)<br>description, paragraphs 44-76 | 21-27 |
| Y | CN 105740799 A (SHENZHEN UNIVERSITY) 06 July 2016 (2016-07-06)<br>description, paragraphs 23-117 | 21-27 |

☑ Further documents are listed in the continuation of Box C. ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| <div align="center">**23 May 2023**</div> | <div align="center">**15 June 2023**</div> |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2023/085278** |

**C.     DOCUMENTS CONSIDERED TO BE RELEVANT**

| Category* | Citation of document, with indication, where appropriate, of the relevant passages | Relevant to claim No. |
|---|---|---|
| X | CN 112881351 A (SHENZHEN DYMIND BIOTECHNOLOGY CO., LTD.) 01 June 2021 (2021-06-01)<br>       description, paragraphs 27-102 | 28 |
| X | CN 104977238 A (SHENZHEN MINDRAY BIO-MEDICAL ELECTRONICS CO., LTD. et al.) 14 October 2015 (2015-10-14)<br>       description, paragraphs 37-69 | 28 |
| Y | CN 111785363 A (CIXI INSTITUTE OF BIOMEDICAL ENGINEERING, NINGBO INSTITUTE OF INDUSTRIAL TECHNOLOGY, CHINESE ACADEMY OF SCIENCES et al.) 16 October 2020 (2020-10-16)<br>       description, paragraphs 19-29 | 19 |
| Y | CN 107194158 A (SHENZHEN MGENE TECHNOLOGY CO., LTD.) 22 September 2017 (2017-09-22)<br>       description, paragraphs 50-139 | 19 |
| Y | CN 112881351 A (SHENZHEN DYMIND BIOTECHNOLOGY CO., LTD.) 01 June 2021 (2021-06-01)<br>       description, paragraphs 27-102 | 19 |
| Y | CN 104977238 A (SHENZHEN MINDRAY BIO-MEDICAL ELECTRONICS CO., LTD. et al.) 14 October 2015 (2015-10-14)<br>       description, paragraphs 37-69 | 19 |
| A | US 2013226611 A1 (KEIMYUNG UNIVERSITY INDUSTRY ACADEMIC COOPERATION FOUNDATION) 29 August 2013 (2013-08-29)<br>       entire document | 1-31 |

Form PCT/ISA/210 (second sheet) (July 2022)

INTERNATIONAL SEARCH REPORT
Information on patent family members

| | International application No. |
|---|---|
| | **PCT/CN2023/085278** |

| Patent document cited in search report | | | Publication date (day/month/year) | Patent family member(s) | | | Publication date (day/month/year) |
|---|---|---|---|---|---|---|---|
| CN | 111785363 | A | 16 October 2020 | None | | | |
| CN | 107194158 | A | 22 September 2017 | None | | | |
| CN | 110911009 | A | 24 March 2020 | None | | | |
| CN | 109346169 | A | 15 February 2019 | None | | | |
| CN | 105740799 | A | 06 July 2016 | US | 2018268195 | A1 | 20 September 2018 |
| | | | | US | 10783371 | B2 | 22 September 2020 |
| | | | | WO | 2017128799 | A1 | 03 August 2017 |
| | | | | CN | 105740799 | B | 16 February 2018 |
| CN | 112881351 | A | 01 June 2021 | None | | | |
| CN | 104977238 | A | 14 October 2015 | CN | 104977238 | B | 16 February 2018 |
| US | 2013226611 | A1 | 29 August 2013 | KR | 20120107750 | A | 04 October 2012 |
| | | | | KR | 101224135 | B1 | 21 January 2013 |
| | | | | WO | 2012128435 | A1 | 27 September 2012 |

Form PCT/ISA/210 (patent family annex) (July 2022)